# EUROPEAN PATENT APPLICATION

(11) **EP 4 549 544 A1**
(43) Date of publication of application: **07.05.2025**
(21) Application number: 23831623.6
(22) Date of filing: 30.06.2023
(51) Int. Cl.: C12M 1/00, C12P 7/00, C12P 7/06

(54) **PRODUCTION SYSTEM FOR ORGANIC SUBSTANCE, PRODUCTION DEVICE FOR ORGANIC SUBSTANCE, AND METHOD FOR PRODUCING ORGANIC SUBSTANCE**

(30) Priority: 30.06.2022 JP 2022106574; 30.06.2022 JP 2022106575; 30.06.2022 JP 2022106142
(71) Applicant: SEKISUI CHEMICAL CO., LTD., Osaka-shi Osaka 530-8565 (JP)
(72) Inventor: KAWAI, Hiroshi, Tokyo 105-8566 (JP); HIGASHI, Eiji, Tokyo 105-8566 (JP); SATOU, Kanetomo, Tsukuba-shi, Ibaraki 300-4292 (JP); MIKI, Nanami, Tsukuba-shi, Ibaraki 300-4292 (JP); NOMURA, Shun, Tokyo 105-8566 (JP)
(74) Representative: Ter Meer Steinmeister & Partner
(86) International application number: PCT/JP2023/024356
(87) International publication number: WO 2024/005184

(57) **Abstract**

[Problem] To provide a production system for an organic substance that can improve the organic substance production efficiency.

[Solution] According to one aspect of the present disclosure, there is provided a production system for an organic substance. The production system includes a gasification furnace configured to generate an exhaust gas, a first raw material gas generation unit configured to generate a first raw material gas containing carbon monoxide from the exhaust gas, a culture tank to which the first raw material gas is supplied and in which an organic substance-containing solution is generated by the action of gas-utilizing bacteria, a recovery unit configured to recover the organic substance from the organic substance-containing solution supplied from the culture tank, and a treatment unit configured to obtain a second raw material gas containing carbon monoxide from a waste liquid generated upstream of the culture tank.

## Description

### [Technical Field]

The present disclosure relates to a production system for an organic substance, a production device for an organic substance and a method for producing an organic substance.

### [Background Art]

In recent years, the mass consumption of oils, alcohols, and so on, which are produced using petroleum as a raw material has led to concerns about the depletion of fossil fuel resources and global environmental problems such as an increase in carbon dioxide in the atmosphere. In order to address these problems, methods for producing organic substances using raw materials other than petroleum, for example, a method for producing bioethanol from an edible raw material such as corn using a sugar fermentation method, have been focused on.

Such a sugar fermentation method using an edible raw material has a risk of raising food prices because limited agricultural land areas are used to produce non-food products.

Therefore, methods for producing organic substances using non-edible raw materials that would have been discarded in the past are being studied.

For example, PTL 1 discloses a technique for producing ethanol from a gas containing carbon monoxide and hydrogen according to a biochemical conversion and a catalyst reaction.

In addition, PTL 2 and 3 each disclose a method for converting a raw material gas containing carbon dioxide, carbon monoxide and hydrogen into ethanol using gas-utilizing bacteria.

### [Citation List]

### [Patent Literature]

[PTL 1] Japanese Patent Application Publication No. 2012-205530
[PTL 2] Japanese Translation of PCT Application No. 2020-532990
[PTL 3] Japanese Patent Application Publication No. 2018-070465

### [Summary of Invention]

### [Technical Problem]

However, there is also a demand for further improvement in resource efficiency when an organic substance is produced.

In view of the above circumstances, one aspect provides, for example, a production system for an organic substance that can improve the organic substance production efficiency.

In addition, according to PTL 2, when the concentration of hydrogen contained in the raw material gas is high, long-term selectivity and stability of organic substances are often insufficient.

In view of the above circumstances, another aspect provides, for example, a production system for an organic substance that can improve the organic substance production efficiency by separating hydrogen contained in a raw material gas and effectively using the separated hydrogen.

In addition, according to studies by the inventors, the raw material gas contains a considerable amount of nitrogen, and it is difficult to reduce the size of a device used for preprocessing the raw material gas.

In view of the above circumstances, another aspect provides, for example, a production system for an organic substance in which the size of a device configured to process a downstream raw material gas can be reduced by separating nitrogen contained in a raw material gas.

### [Solution to Problem]

According to one aspect of the present disclosure, there is provided a production system for an organic substance. The production system includes a gasification furnace configured to generate an exhaust gas, a first raw material gas generation unit configured to generate a first raw material gas containing carbon monoxide from the exhaust gas, a culture tank to which the first raw material gas is supplied and in which an organic substance-containing solution is generated by the action of gas-utilizing bacteria, a recovery unit configured to recover the organic substance from the organic substance-containing solution supplied from the culture tank, and a treatment unit configured to obtain a second raw material gas containing carbon monoxide from a waste liquid generated upstream of the culture tank.

According to this aspect, there is provided, for example, a production system for an organic substance that can improve organic substance production efficiency.

According to another aspect, there is provided a production system for an organic substance. The production system includes a gas generation unit configured to generate a raw material gas containing carbon monoxide, carbon dioxide and hydrogen, a culture tank to which the raw material gas is supplied and in which an organic substance-containing solution is generated by the action of gas-utilizing bacteria, a first separation unit configured to separate hydrogen from the raw material gas, and a reduction unit having a reductant that reduces the carbon dioxide using the hydrogen separated in the carbon dioxide and the first separation unit to generate carbon monoxide.

According to this aspect, it is possible to produce an organic substance with higher production efficiency.

According to another aspect, there is provided a production system for an organic substance. The production system includes a gas generation unit configured to generate a raw material gas containing carbon monoxide and nitrogen, a separation unit configured to separate nitrogen from the raw material gas, and a culture tank to which the raw material gas from which nitrogen is separated in the separation unit is supplied and in which an organic substance-containing solution is generated by the action of gas-utilizing bacteria.

According to this aspect, it is possible to produce an organic substance in a smaller facility.

### [Brief Description of Drawings]

[Fig. 1]

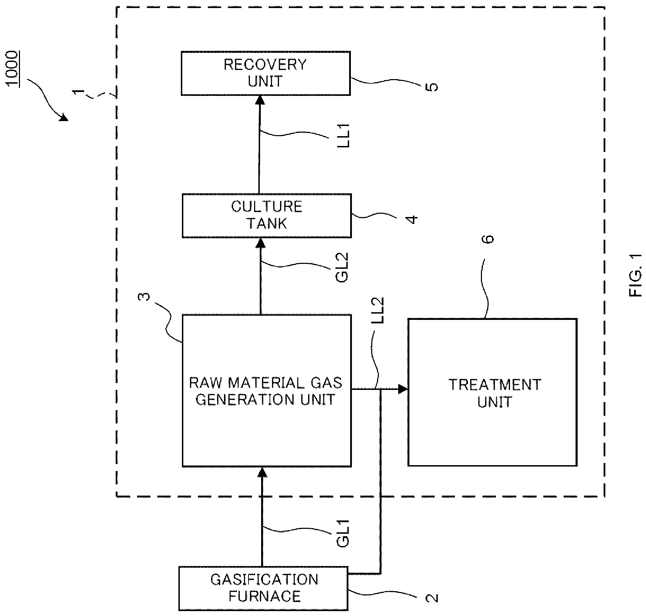

   Fig. 1 is a schematic view showing a configuration of a production system for an organic substance according to Embodiment 1.
[Fig. 2]

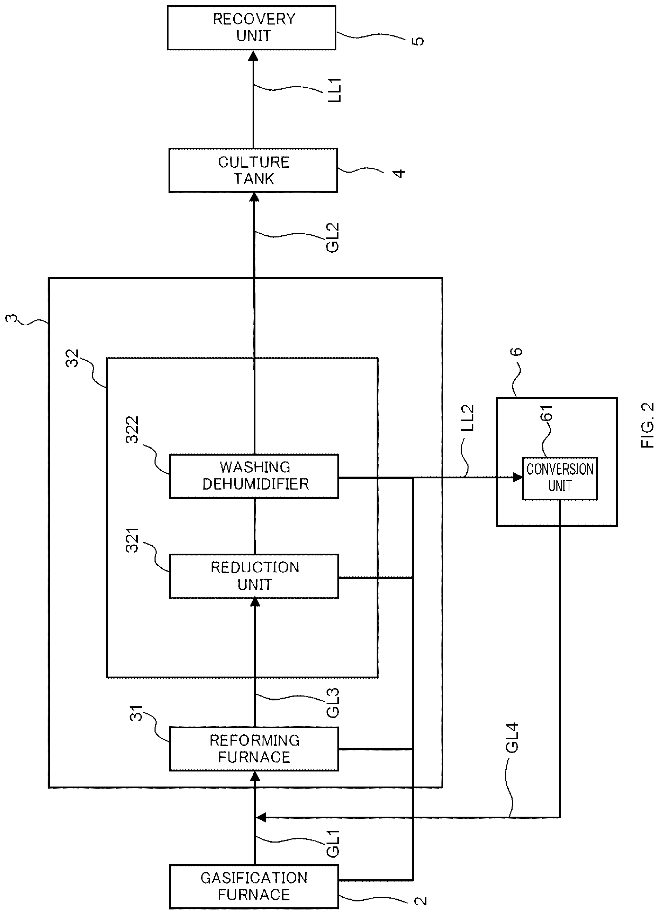

   Fig. 2 is a schematic view showing a configuration of a production system for an organic substance according to Embodiment 2.
[Fig. 3]

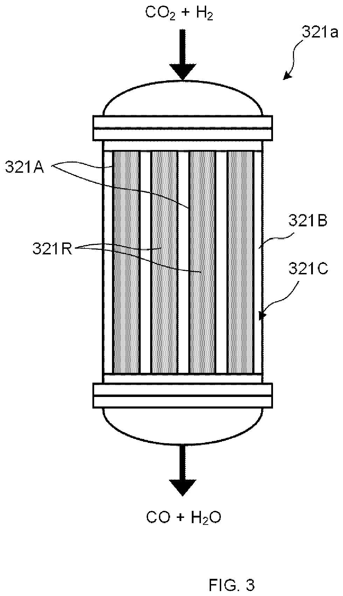

   Fig. 3 is a schematic view showing a configuration of a reduction reactor 321a in the production system for an organic substance.
[Fig. 4]

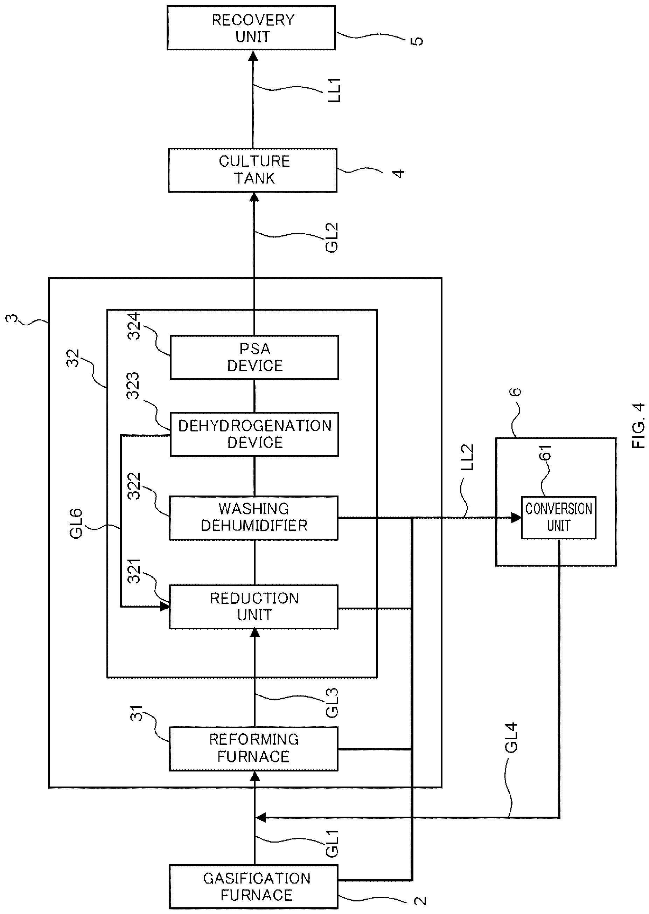

   Fig. 4 is a schematic view showing another configuration of a first purification unit 32 in the production system for an organic substance according to Embodiment 2.
[Fig. 5]

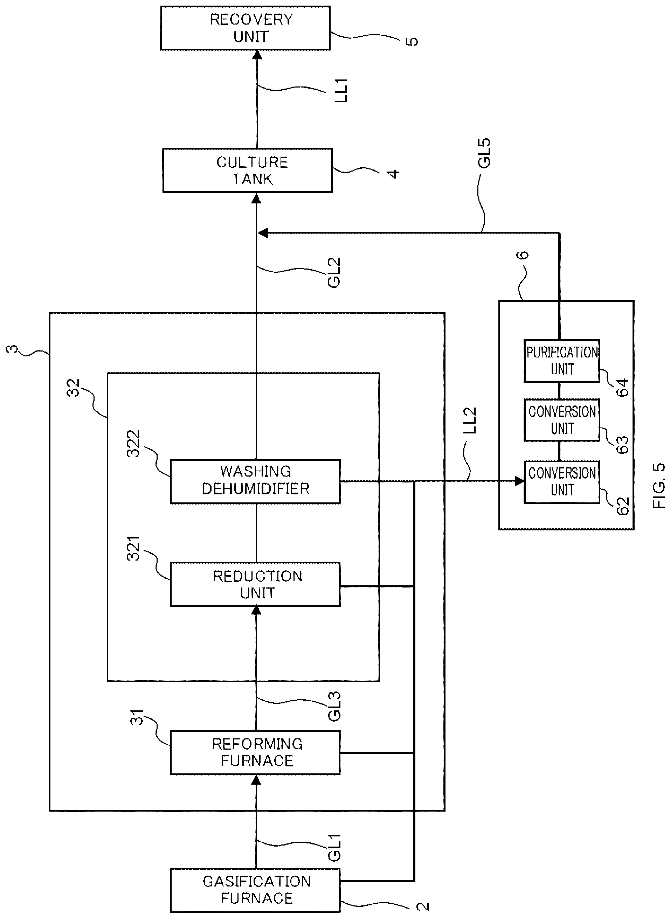

   Fig. 5 is a schematic view showing a configuration of a production system for an organic substance according to Embodiment 3.
[Fig. 6]

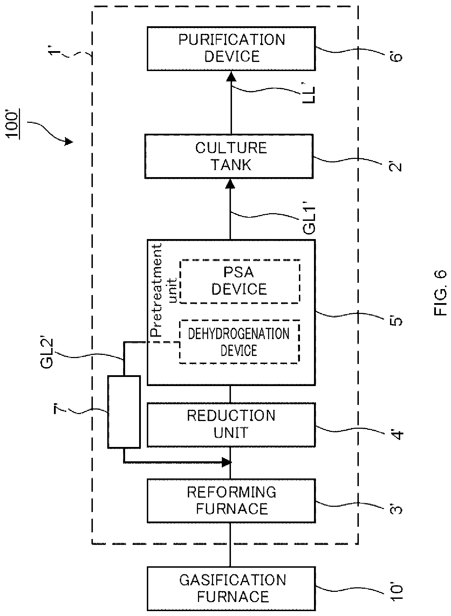

   Fig. 6 is a schematic view showing a configuration of a production system for an organic substance according to Embodiment 1'.
[Fig. 7]

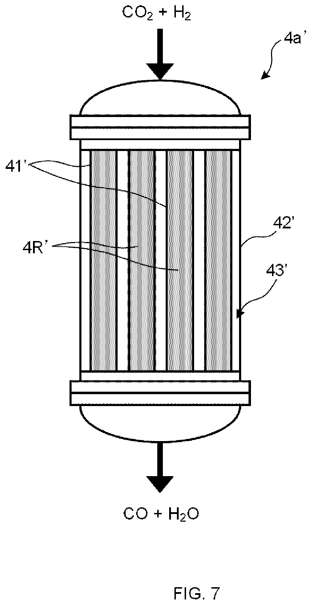

   Fig. 7 is a schematic view showing a configuration of a reduction unit in the production system for an organic substance according to Embodiment 1'.
[Fig. 8]

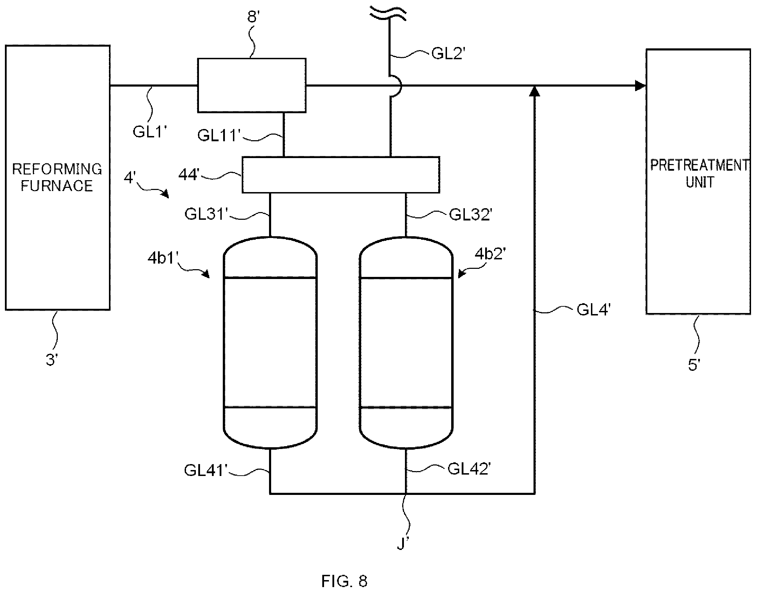

   Fig. 8 is a schematic view showing a configuration of a reduction unit and the vicinity thereof in a production system for an organic substance according to Embodiment 2'.
[Fig. 9]

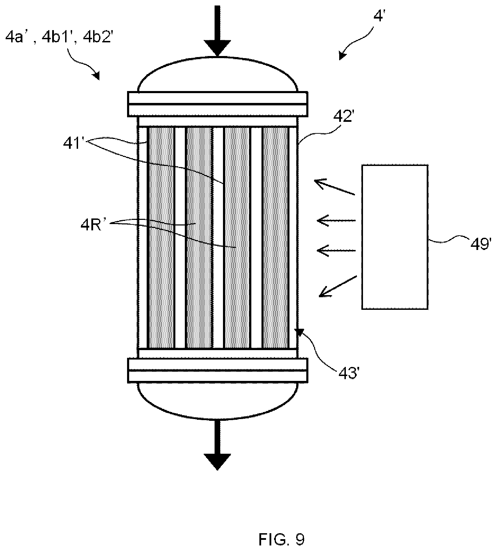

   Fig. 9 is a schematic view showing a configuration of a reduction unit including an emission device that emits microwaves.
[Fig. 10]

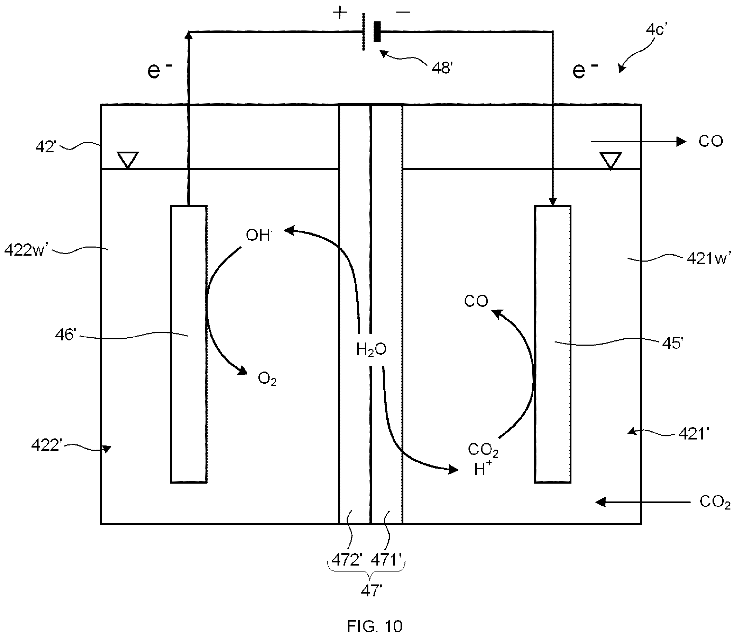

   Fig. 10 is a schematic view showing a configuration of a reduction unit in a production system for an organic substance according to Embodiment 3'.

### [Description of Embodiments]

Hereinafter, a production system for an organic substance, a production device for an organic substance and a method for producing an organic substance will be described in detail with reference to preferable embodiments shown in the appended drawings.

### <Embodiment 1>

First, a production system for an organic substance according to Embodiment 1 will be described with reference to Fig. 1 and the like.

That is, the production system for an organic substance according to Embodiment 1 is shown below.

A production system for an organic substance includes:
a gasification furnace configured to generate an exhaust gas;
a first raw material gas generation unit configured to generate a first raw material gas containing carbon monoxide from the exhaust gas;
a culture tank in which the first raw material gas is supplied and an organic substance-containing solution is generated by the action of gas-utilizing bacteria;
a recovery unit configured to recover the organic substance from the organic substance-containing solution supplied from the culture tank; and
a treatment unit configured to obtain a second raw material gas containing carbon monoxide from a waste liquid generated upstream of the culture tank.

Fig. 1 is a schematic view showing a configuration of a production system for an organic substance according to Embodiment 1 of the present disclosure.

A production system 1000 for an organic substance shown in Fig. 1 (hereinafter simply referred to as "production system 1000") includes a gasification furnace (gas generation unit) 2 and a production device 1 for an organic substance (hereinafter simply referred to as a "production device 1") connected to the gasification furnace 2.

Here, in this specification, the upstream side and the downstream side with respect to the flow direction of a gas and a liquid are simply referred to as "upstream" and "downstream," respectively.

In the present embodiment, the gasification furnace 2 is, for example, a combustion furnace, a blast furnace, a converter furnace, an electric furnace, or a shaft furnace. Alternatively, it may be a COₓ emission source of at least one industrial facility selected from among a paper factory, a cement factory, a thermal power plant, an oil refinery, an ethylene cracker, an oil refinery, and a chemical plant.

In each furnace, when the contents are combusted, melted, and refined, an exhaust gas containing carbon dioxide and carbon monoxide (raw material gas) is generated (produced).

In the case of a combustion furnace (incinerator) in a waste incineration plant, the contents (waste) include, for example, plastic waste, food waste, municipal solid waste (MSW), industrial waste, discarded tires, biomass waste, household waste (futon material, paper), building members, and the like. Here, the contents may contain these wastes alone or two or more thereof.

The exhaust gas generally contains carbon dioxide and carbon monoxide, and may further contain other gas components such as hydrogen, nitrogen, oxygen, water vapor, and methane. The exhaust gas may further contain, as other components, soot, tar, a nitrogen compound, a sulfur compound, a phosphorus compound, an aromatic compound, and the like.

The exhaust gas may be generated as a gas containing 10 vol% or more of carbon monoxide by subjecting the content (carbon source) to an incomplete combustion heat treatment (commonly known as gasification) (that is, partially oxidizing a carbon source).

When the exhaust gas is used, it is possible to effectively use carbon dioxide that would have been discharged into the air in the past, and it is possible to reduce the burden on the environment. Among these, in consideration of carbon circulation, an exhaust gas produced in a combustion furnace or smelter is preferable.

The production device 1 is connected to the gasification furnace 2. The production device 1 includes a raw material gas generation unit 3, a culture tank 4, a recovery unit 5, and a treatment unit 6. In addition, the gasification furnace 2 and the raw material gas generation unit 3 are connected by a gas line GL1, and the raw material gas generation unit 3 and the culture tank 4 are connected by a gas line GL2. In addition, the culture tank 4 and the recovery unit 5 are connected by a liquid line LL1, and the gasification furnace 2 and the raw material gas generation unit 3, and the treatment unit 6 are connected by a liquid line LL2.

The raw material gas generation unit 3 generates a first raw material gas containing carbon monoxide.

That is, the raw material gas generation unit 3 processes the exhaust gas discharged from the gasification furnace 2 and generates a raw material gas suitable for use in the culture tank 4. This process is not particularly limited, and more specific details will be described in the section of Embodiment 2.

In the culture tank 4, an organic substance-containing solution is generated from the supplied first raw material gas by the action of gas-utilizing bacteria. Here, the action of gas-utilizing bacteria is, for example, microbial fermentation.

In addition, gas-utilizing bacteria include both eubacteria and archaebacteria.

Examples of eubacteria include Clostridium bacteria, Moorella bacteria, Acetobacterium bacteria, Carboxydocella bacteria, Rhodopseudomonas bacteria, Eubacterium bacteria, Butyribacterium bacteria, Oligotropha bacteria, Bradyrhizobium bacteria, and Ralsotonia bacteria which is aerobic hydrogen-oxidizing bacteria.

On the other hand, examples of archaebacteria include Methanobacterium bacteria, Methanobrevibacter bacteria, Methanocalculus bacteria, Methanococcus bacteria, Methanosarcina bacteria, Methanosphaera bacteria, Methanothermobacter bacteria, Methanothrix bacteria, Methanoculleus bacteria, Methanofollis bacteria, Methanogenium bacteria, Methanospirillium bacteria, Methanosaeta bacteria, Thermococcus bacteria, Thermofilum bacteria, and Arcaheoglobus bacteria.

Among the above gas-utilizing bacteria, bacteria with a high production capacity of a desired organic substance are selected and used. Examples of gas-utilizing bacteria with a high ethanol production capacity include Clostridium autoethanogenum, Clostridium ljungdahlii, Clostridium aceticum, Clostridium carboxidivorans, Moorella thermoacetica, and Acetobacterium woodii.

A medium (culture solution) used for culturing gas-utilizing bacteria is not particularly limited as long as it has an appropriate composition according to the type of bacteria. For example, when Clostridium bacteria are used as gas-utilizing bacteria, as the medium, for example, paragraph 0091 in WO 2017/117309, and paragraphs 0097 and 0098 in U.S. Patent Application Publication No. 2017/260552 can be referred to.

For the culture tank 4, for example, a culture reactor type configured to stir a culture solution with a stirring plate, a culture reactor type configured to stir a culture solution by circulating the culture solution itself, a culture reactor type configured to stir a culture solution by a water flow caused by a bubble flow occurring due to the aeration of a supplied exhaust gas, and the like can be used.

The concentration of carbon monoxide contained in the raw material gas supplied to the culture tank 4 is preferably 10 vol% or more and 80 vol% or less, more preferably 15 vol% or more and 50 vol% or less, and still more preferably 20 vol% or more and 45 vol% or less.

In addition, the concentration of hydrogen contained in the raw material gas supplied to the culture tank 4 is preferably 1 vol% or more and 45 vol% or less, more preferably 1 vol% or more and 40 vol% or less, still more preferably 5 vol% or more and 35 vol% or less, and particularly preferably 10 vol% or more and 30 vol% or less.

In addition, the concentration of nitrogen contained in the raw material gas supplied to the culture tank 4 is preferably 30 vol% or less, more preferably 1 vol% or more and 25 vol% or less, and still more preferably 5 vol% or more and 20 vol% or less.

According to the above configuration, since an exhaust gas with a low hydrogen concentration is supplied to the culture tank 4, hydrogen is less likely to reduce the activity of gas-utilizing bacteria.

In the culture tank 4, an organic substance-containing solution is generated from the raw material gas by the action of gas-utilizing bacteria, the recovery unit 5 is connected to the culture tank 4 through the liquid line LL1, and the recovery unit 5 recovers the organic substance from the organic substance-containing solution supplied from the culture tank 4. The recovery unit 5 is typically a device configured to purify and recover the organic substance from the organic substance-containing solution.

Examples of the recovery unit 5 include a distillation device, a processing device including an osmotic evaporating membrane, a processing device including a zeolite dehydration membrane and an organic membrane, a processing device configured to remove a low-boiling-point substance that has a lower boiling point than the organic substance, a processing device configured to remove a high-boiling-point substance that has a higher boiling point than the organic substance, and a processing device including an ion exchange membrane. These devices may be used alone or two or more thereof may be used in combination.

When a distillation device is used, the temperature in the distillation device during distillation of the organic substance (particularly, ethanol) is not particularly limited, and is preferably 100°C or lower and more preferably 70°C or higher and 95°C or lower. When the temperature is set at such a level, the separation of a necessary organic substance from other components, that is, distillation (purification) of an organic substance, can be performed more reliably.

The pressure in the distillation device during distillation of the organic substance may be atmospheric pressure, and is preferably less than atmospheric pressure (reduced pressure distillation) and more preferably 60 kPaG or more and 95 kPaG or less. When the pressure is set at such a level, it is possible to improve the organic substance separation efficiency and thus it is possible to improve the yield of the organic substance.

The yield of the organic substance (concentration of the organic substance contained in the purified product) is preferably 90 wt% or more, more preferably 99 wt% or more, and still more preferably 99.5 wt% or more.

Examples of organic substances obtained in this manner include monools such as methanol and ethanol, diols such as 2,3-Butanediol, acetic acid, lactic acid, isoprene, and butadiene, and monools or diols having 1 to 4 carbon atoms are preferable, and ethanol is more preferable.

Such organic substances can be used, for example, as raw materials such as resin materials and rubber materials, and can be used as various solvents, disinfectants or fuels. Here, high-concentration ethanol can be used as fuel ethanol to be mixed with gasoline or the like, and can be used, for example, as a raw material for cosmetics, beverages, chemical substances, and fuels (jet fuels), and an additive for foods and the like, and is extremely versatile.

In addition, the production system 1000 includes the treatment unit 6 configured to obtain a second raw material gas containing carbon monoxide from the waste liquid generated upstream of the culture tank 4. The treatment unit 6 is connected to the gasification furnace 2 and the raw material gas generation unit 3 through the liquid line LL2, and is configured to be able to supply the waste liquid generated in each of these facilities.

Here, the type of the waste liquid generated in these facilities is not particularly limited and for example, it contains an organic component (including soot during heating) derived from the waste put into the gasification furnace 2. Here, the waste liquid supplied to the treatment unit 6 is not limited to the waste liquid generated during the operation of the production system 1000 and may also include waste liquid generated during maintenance and the like.

In addition, the waste liquid may also include a waste liquid discharged from an ancillary facility (for example, a laboratory and an office) of an industrial facility including the gasification furnace 2.

In addition, the treatment unit 6 is configured to perform various treatments on the supplied waste liquid to obtain a second raw material gas containing carbon monoxide. The treatments include a heat treatment, a mechanical treatment, a chemical and/or biological treatment, but the present disclosure is not limited thereto. In addition, typical examples will be described in Embodiment 2 and Embodiment 3.

Here, the second raw material gas containing carbon monoxide may be subsequently used in the production device 1, and may be used in another industrial process and the like independently of the production device 1. In a preferable embodiment, a mixed gas of the first raw material gas and the second raw material gas can be generated and then supplied to the culture tank 4, which can contribute to improving the amount of the organic substance produced. Here, the first raw material gas and the second raw material gas may not be mixed but may each be supplied to the culture tank 4 through a separate path.

Next, a method of using the production system 1000 according to Embodiment 1 (a method for producing an organic substance) will be described.
[1A] First, the exhaust gas (typically, a gas containing carbon monoxide, carbon dioxide, hydrogen, and other gas components) discharged from the gasification furnace 2 is supplied to the raw material gas generation unit 3. In this case, the exhaust gas is converted to a first raw material gas containing carbon monoxide (first raw material gas generation step).
[2A] Next, the raw material gas containing carbon monoxide is supplied to the culture tank 4. In the culture tank 4, an organic substance-containing solution is generated from the raw material gas by the action of gas-utilizing bacteria (culture step).
[3A] The organic substance-containing solution generated in the culture tank 4 is supplied to the recovery unit 5 through the liquid line LL1. The recovery unit 5 recovers the organic substance contained in the organic substance-containing solution (recovery step). In the recovery unit 5, typically, the organic substance-containing solution is purified, and a purified product containing the organic substance at a high concentration is obtained.
[4A] On the other hand, the waste liquid generated in the raw material gas generation unit 3 is supplied to the treatment unit 6. In the treatment unit 6, a second raw material gas containing carbon monoxide is obtained from the waste liquid generated upstream of the culture tank 4 (treatment step). The second raw material gas can be used in various industrial processes, and as an example, a mixed gas of the first raw material gas and the second raw material gas can be generated and then supplied to the culture tank 4.

While the configuration and usage method of the production system 1000 according to Embodiment 1 have been described above, in the production system 1000, the waste liquid generated in the raw material gas generation unit 3 can be used, and thus it can be said that the organic substance production efficiency can be improved.

### <Embodiment 2>

Next, a production system for an organic substance according to Embodiment 2 will be described with reference to Fig. 2 and the like.

Fig. 2 is a schematic view showing a configuration of the production system for an organic substance according to Embodiment 2.

In the production system for an organic substance according to Embodiment 2 shown in Fig. 2, the raw material gas generation unit 3 includes a reforming furnace 31 and a first purification unit 32. Here, the reforming furnace 31 converts at least some of methane contained in the exhaust gas to carbon monoxide to generate a first crude gas. In addition, the first purification unit 32 purifies the first crude gas to generate a first raw material gas.

That is, in the production system for an organic substance according to Embodiment 2, a more specific configuration of the raw material gas generation unit 3 is shown. Here, as shown in Fig. 2, the first purification unit 32 includes a reduction unit 321 and a washing dehumidifier 322, but the production system for an organic substance does not need to include all of these components, and other components can also be added as necessary.

In addition, in the production system for an organic substance according to Embodiment 2, the treatment unit 6 obtains a second raw material gas from the waste liquid generated in the reforming furnace 31 and/or the first purification unit 32. In addition, the treatment unit 6 includes a first conversion unit 61 in addition to the raw material gas generation unit 3. The first conversion unit 61 converts at least a part of the waste liquid to methane. Then, the methane converted by the first conversion unit 61 is transferred to the reforming furnace 31.

That is, in Embodiment 2, methane is obtained from the waste liquid by the first conversion unit 61 and returns to the reforming furnace 31 and is converted to carbon monoxide. In other words, the reforming furnace 31 is a part of the raw material gas generation unit 3, and at the same time, is a part of the treatment unit 6.

Hereinafter, the production system for an organic substance according to Embodiment 2 will be described, focusing on the differences from the production system for an organic substance according to Embodiment 1, and the same points will not be described.

The exhaust gas discharged from the gasification furnace 2 is transferred to the reforming furnace 31 through the gas line GL1.

In the reforming furnace 31, for example, in the presence of a catalyst, methane contained in the exhaust gas is reacted with water vapor at a high temperature and is converted to carbon monoxide and hydrogen. In this case, some of the carbon monoxide may be additionally converted to carbon dioxide and hydrogen by reacting with water vapor.

The reaction temperature is preferably 500°C or higher and 1,100°C or lower. In addition, examples of catalysts include a nickel catalyst, a nickel oxide catalyst, a ruthenium catalyst, a rhodium catalyst, a palladium catalyst, and a platinum catalyst.

The reforming furnace 31 may execute a method for converting gases such as methane and ethane contained in the exhaust gas and hydrocarbons such as tar and dioxin to carbon monoxide and hydrogen by retaining them at a high temperature. In this case, a high temperature condition may be set after a combustible gas such as oxygen or air is supplied. In addition, some of the carbon monoxide may be converted to carbon dioxide by reacting with oxygen. In this case, the temperature is preferably 1,000°C or higher and more preferably 1,100°C or higher and 1,300°C or lower.

Here, in the production system for an organic substance in Fig. 2, the gasification furnace 2 and the reforming furnace 31 are shown as independent components, but the composition of the exhaust gas can be appropriately controlled using a furnace integrally including a gasification region (gasification zone) and a reforming region (reforming zone) such as a thermoselect gasification and reforming furnace. In this case, as an example, the composition of the exhaust gas can be appropriately controlled by returning the gas produced from the thermoselect gasification and reforming furnace to the reforming zone again.

The exhaust gas passed through the reforming furnace 31 is supplied to a reduction reactor 321a (the reduction unit 321) through a gas line GL3. In the reduction reactor of the present embodiment, carbon monoxide can be generated from carbon dioxide (carbon dioxide can be converted to carbon monoxide) using the hydrogen according to a reverse water gas shift reaction caused by the action of a catalyst.

Fig. 3 is a schematic view showing a configuration of the reduction reactor 321a in the production system for an organic substance.

As shown in Fig. 3, the reduction reactor 321a is a multi-tubular reaction device (fixed bed type reaction device) including a plurality of tubes 321A each filled with (containing) a catalyst 321R and a housing 321C in which the plurality of tubes 321A are stored in an internal space 321B. According to such a multi-tubular reaction device, it is possible to secure sufficient opportunities for contact between the catalyst 321R and the gas. As a result, it is possible to increase the efficiency of conversion of carbon dioxide to carbon monoxide.

Here, the reduction reactor 321a may be a reactor (that is, a simple reactor) configured by omitting the tube 321A and filling the internal space 321B of the housing 321C with the catalyst 321R.

The catalyst 321R of the present embodiment is preferably in the form of, for example, particles (granules), flakes, or pellets. With the catalyst 321R having such a shape, it is possible to increase the efficiency of filling the tube 321A, and it is possible to further increase the area in contact with the exhaust gas supplied into the tube 321A.

When the catalyst 321R is in the form of particles, the volume average particle size thereof is not particularly limited, and is preferably 1 mm or more and 50 mm or less and more preferably 1 mm or more and 30 mm or less. In this case, it is possible to further increase the contact area between the catalyst 321R and the gas (carbon dioxide, hydrogen, etc.) and it is possible to further improve the efficiency of conversion of carbon dioxide to carbon monoxide.

The particulate catalyst 321R is preferably a molded component produced by rolling granulation because this further increases the sphericity.

In addition, the catalyst 321R may be supported on a carrier.

The material constituting the carrier may be any material that is not easily denatured by the contact with the exhaust gas, reaction conditions and the like, and examples thereof include carbon materials (graphite, graphene, carbon black, carbon nanotubes, activated carbon, etc.), carbides such as Mo₂C, oxides such as zeolite, montmorillonite, ZrO₂, TiO₂, V₂O₅, MgO, CeO₂, Al₂O₃, and SiO₂, and composite oxides containing these.

Among these, zeolite, montmorillonite, ZrO₂, TiO₂, V₂O₅, MgO, Al₂O₃, SiO₂ and a composite oxide containing these are preferable as the material constituting the carrier. A carrier made of such a material is preferable because it does not adversely affect the reaction of the catalyst 321R and has an excellent ability to support the catalyst 321R. Here, the carrier does not participate in the reaction of the catalyst 321R but simply supports (holds) the catalyst 321R.

As an example of such a configuration, a configuration in which at least a part of the surface of the carrier is covered with the catalyst 321R may be exemplified.

The catalyst 321R includes a transition metal. The transition metal is not particularly limited as long as it can reduce carbon dioxide, and examples thereof include Fe, Sc, Ti, V, Cr, Mn, Co, Ni, Cu, Zn, Y, Zr, Nb, Mo, Tc, Ru, Rh, Pd, Ag, Cd, La, Ce, Pr, Nd, Pm, Sm, Eu, Gd, Tb, Dy, Ho, Er, Tm, Yb, Lu, Hf, Ta, W, Re, Os, Ir, Pt, Au, and Hg.

The catalyst 321R is preferably an alloy obtained by adding at least one metal selected from the group consisting of Al, Ga, In, Cu, Ag, Au, Pd and Mn to Fe and more preferably an Fe-Pd alloy. These catalysts 321R are useful because they have particularly favorable efficiency of conversion of carbon dioxide to carbon monoxide.

In addition, in the reduction reactor 321a, the catalyst 321R itself may be used to prepare the tube (cylindrical molded component) 321A. In addition, the catalyst 321R may be used to prepare a block or lattice (for example, mesh or honeycomb) molded component and disposed in the housing 321C. In these cases, the catalyst 321R as a filling agent may be omitted or may be used in combination.

Among these, a configuration in which a mesh component is prepared using the catalyst 321R and disposed in the housing 321C is preferable. In such a configuration, it is possible to prevent an increase in the resistance of the exhaust gas passing through the reduction reactor 321a and sufficiently secure opportunities for contact between the catalyst 321R and the exhaust gas.

In addition, the first purification unit 32 includes the washing dehumidifier 322.

The washing dehumidifier 322 is a so-called scrubber, and is used to remove pollutants (for example, soot), watersoluble substances, and the like contained in the exhaust gas. In the washing dehumidifier 322, the removal is performed by bringing a washing solution into contact with an object to be removed (wet washing method). As an example of the wet washing method, a washing method using a water curtain may be exemplified. In addition, examples of washing solutions include water, an acidic solution, and an alkaline solution. Among these, water is preferable as the washing solution. In addition, the temperature of the washing solution is generally 40°C or lower, preferably 30°C or lower, more preferably 25°C or lower, and still more preferably 15°C or lower.

Fig. 4 is a schematic view showing another configuration of the first purification unit 32 in the production system for an organic substance according to Embodiment 2.

In the first purification unit 32, the first purification unit 32 may further include a PSA device 324 or a TSA device.

The PSA device 324 is a pressure swing adsorption type separator and is used to remove (separate), for example, BTEX (benzene, toluene, ethylbenzene, xylene), carbon dioxide, nitrogen, and the like from the first crude gas. When the PSA device 324 removes nitrogen, it constitutes a nitrogen separation unit. In the configuration shown in Fig. 4, the PSA device 324 is positioned (connected) downstream of the reduction reactor 321a (the reduction unit 321).

The TSA device is a temperature swing adsorption type separator, and is used to remove, for example, an aromatic compound other than BTEX.

In the PSA device 324 and the TSA device, for example, porous materials such as activated carbon, zeolite, silica gel, and molecular sieves, and aqueous solutions such as an amine solution can be used as adsorbents. In the PSA device 324 and the TSA device, activated carbon or zeolite is preferably used. When the type of the porous material and the size of pores are set, it is possible to select a compound that can be separated.

When two or more compounds in the PSA device 324 and the TSA device are separated, a plurality of separators each filled with porous materials with different types and pore sizes may be used or a single separator filled with porous materials with different types and pore sizes may be used.

The carbon dioxide separated in the PSA device 324 may be mixed with the exhaust gas and supplied to the reduction unit 321.

In addition, the nitrogen separated in the PSA device 324 may be filled into the culture tank 4 when gas-utilizing bacteria are cultured, filled for washing the reduction unit 321, filled for washing the PSA device 324 and/or the TSA device, filled for washing a deoxidation device (if used) and/or deacetylation device (if used), filled for washing pipes in a gas analysis facility, filled for sealing a culture solution storage tank to prevent oxidation of valuables, filled for sealing the purified valuable to prevent oxidation, or filled for washing the facility in a gasification furnace process. Here, nitrogen may be filled into only one of the above devices or filled into two or more of the above devices.

In this manner, when nitrogen is removed from the exhaust gas, the volume of the exhaust gas to be treated downstream can be reduced, and thus the size of the first purification unit 32 disposed downstream can be reduced.

In addition, the first purification unit 32 may include a filter.

The filter is used to remove fine particles having a smaller size than soot. This filter may be, for example, a bag filter.

In addition, the first purification unit 32 may include a device for removing various gases. Examples of such devices include a dehydrogenation device 323, a deoxidation device, and a deacetylation device.

The dehydrogenation device (hydrogen separation unit) 323 is typically positioned (connected) downstream of the reduction reactor 321a (the reduction unit 321) and is used to remove (separate) hydrogen from the first crude gas. The dehydrogenation device 323 may be composed of a separator containing a cylindrical separation membrane that selectively permeates and separates hydrogen.

Examples of materials constituting such a separation membrane include a metal material, a ceramic material, and a resin material.

Examples of metal materials include Pd-Cu alloys, Pd-Ag alloys, vanadium alloys, and amorphous alloys such as La-Ni-Mg alloys.

Examples of ceramic materials include titanium nitride, zeolite, silica (glass), alumina and composite materials containing one or more of these ceramics (for example, alumina carbon-based materials).

Examples of resin materials include polyamides, polyimides, and polysulfones.

The separation membrane is preferably composed of a porous component having continuous pores (pores penetrating the cylindrical wall) in which adjacent pores are connected to each other. A separation membrane having such a configuration can separate hydrogen more smoothly and reliably.

The porosity of the separation membrane is not particularly limited, and is preferably 10% or more and 90% or less and more preferably 20% or more and 60% or less. Thereby, it is possible to prevent the mechanical strength of the separation membrane from decreasing extremely and to maintain a sufficiently high hydrogen permeability.

Here, the shape of the separation membrane is not particularly limited, and examples thereof include angular cylindrical shapes such as a cylindrical shape, a rectangular shape, and a hexagonal shape.

The average pore size of the separation membrane is preferably 500 pm or less and more preferably 300 pm or more and 400 pm or less. Thereby, it is possible to further improve the hydrogen separation efficiency.

Here, the hydrogen separated by the dehydrogenation device 323 may be used for various applications. For example, in the configuration shown in Fig. 4, the dehydrogenation device 323 is connected to the reduction reactor 321a (the reduction unit 321) through a gas line GL6, and supplies the hydrogen separated in the dehydrogenation device to the reduction reactor 321a for use in the reduction reaction. That is, the reduction reactor 321a (the reduction unit 321) reduces carbon dioxide in the first crude gas using the separated hydrogen to generate carbon monoxide.

Here, when the hydrogen separated in the dehydrogenation device in this manner is used in the reduction reaction, it is preferable to use it after impurities are removed.

Such impurities are not particularly limited, and examples thereof include sulfur or sulfur compounds, chlorine or chlorine compounds, and cyanide compounds. Among these, it is preferable to remove sulfur compounds, particularly, hydrogen sulfide, as impurities. By removing hydrogen sulfide, it is possible to effectively prevent the reactivity of the catalyst 321R from extremely decreasing or being deactivated.

Such impurities can be removed, for example, in a reactor filled with a desulfurizing agent. Examples of desulfurizing agents include an iron oxide-based desulfurizing agent, an activated carbon-based desulfurizing agent, a copper-zinc-based desulfurizing agent, a copper-zinc-aluminum-based desulfurizing agent, and a lime-based desulfurizing agent.

The deoxidation device is used to remove oxygen, and may be composed of a reactor filled with particles of a metal, for example, copper (Cu), platinum (Pt), nickel (Ni), and palladium (Pt) as an oxygen removal catalyst. The oxygen removal catalyst is preferably heated to, for example, 150°C or higher and 400°C or lower.

The deacetylation device is used to remove acetylene, and may be composed of a reactor filled with particles of a precious metal, for example, palladium (Pd) and platinum (Pt), as an acetylene removal catalyst.

Here, it is also preferable to remove acetylene before the above deoxidation is performed. In this case, it is possible to effectively prevent or reduce the adverse effect of acetylene on the oxygen removal catalyst.

The exhaust gas (first raw material gas) purified by the above first purification unit 32 is supplied to the culture tank 4 through the gas line GL2.

The concentration of carbon dioxide contained in the exhaust gas (first raw material gas) supplied to the culture tank 4 is preferably 0.1 vol% or more and 9 vol% or less, more preferably 0.3 vol% or more and 8 vol% or less, still more preferably 0.5 vol% or more and 7 vol% or less, particularly preferably 0.8 vol% or more and 6 vol% or less, and most preferably 1 vol% or more and 5 vol% or less.

Similar to Embodiment 1, an organic substance-containing solution is obtained in the culture tank 4, and the organic substance is then recovered by the recovery unit 5.

In addition, in the production system for an organic substance according to Embodiment 2, as described above, the treatment unit 6 obtains a second raw material gas from the waste liquid generated in the reforming furnace 31 and/or the first purification unit 32.

That is, in addition to the gasification furnace 2, the liquid line LL2 is connected to each of components: the reforming furnace 31 and the first purification unit 32, and the waste liquid generated in each component can be supplied to the first conversion unit 61 of the treatment unit 6.

The first conversion unit 61 can convert at least a part of the waste liquid to methane.

In a typical example, the first conversion unit 61 can perform an anaerobic waste liquid treatment (methane fermentation) on the supplied waste liquid. The type of anaerobic bacteria used is not particularly limited, and examples of such anaerobic bacteria include Clostridium bacteria such as Clostridium autoethanogenum, Clostridium ljungdahlii, Clostridium aceticum, Clostridium carboxidivorans, and Clostridium ragsdalei, Moorella bacteria such as Moorella thermoacetica, Acetobacterium bacteria such as Acetobacterium woodii, Carboxydocella bacteria such as Carboxydocella sporoducens sp. nov., Rhodopseudomonas bacteria such as Rhodopseudomonas gelatinosa, Eubacterium bacteria such as Eubacterium limosum, and Butyribacterium bacteria such as Butyribacterium methylotrophicum.

Then, methane converted by the first conversion unit 61 is transferred to the reforming furnace 31 through a gas line GL4 (and the gas line GL1).

That is, in Embodiment 2, methane is obtained from the waste liquid by the first conversion unit 61, returns to the reforming furnace 31, and is converted to carbon monoxide. In other words, it can be said that the reforming furnace 31 is a part of the raw material gas generation unit 3 and at the same time, functions as a part of the treatment unit 6.

Next, a method of using a production system 100 according to Embodiment 2 (a method for producing an organic substance) will be described.
[1B] First, the exhaust gas discharged from the gasification furnace 2 is supplied to the reforming furnace 31.
[2B] Then, the exhaust gas passed through the reforming furnace 31 is supplied to the reduction unit 321 through the gas line GL3. In the reduction reactor 321a, which is an example of the reduction unit 321, the catalyst 321R reduces carbon dioxide by the contact with carbon dioxide and converts it to carbon monoxide. In this case, the catalyst 321R is oxidized by the contact with carbon dioxide.

Here, the temperature of the reduction reactor 321a (exhaust gas, the catalyst 321R) is preferably 125°C or higher and 500°C or lower, more preferably 150°C or higher and 450°C or lower, and still more preferably 175°C or higher and 400°C or lower.

In addition, the pressure of the reduction reactor 321a (exhaust gas, the catalyst 321R) is preferably less than 1 MPaG, more preferably 0.9 MPaG or less, and still more preferably 0.2 MPaG or more and 0.8 MPaG or less.

When the reaction conditions are set within the above ranges, for example, it is possible to proceed the reduction reaction of carbon dioxide in the reduction reactor 321a more smoothly.

[3B] Next, the exhaust gas passed through the reduction unit 321 is supplied to the washing dehumidifier 322.
[4B] The exhaust gas passed through the washing dehumidifier 322 is supplied to the culture tank 4 as a first raw material gas.
[5B] Finally, the organic substance-containing solution generated in the culture tank 4 is supplied to the recovery unit 5.
[6B] On the other hand, the waste liquid generated in the reforming furnace 31 and/or the first purification unit 32 is supplied to the treatment unit 6 through the liquid line LL2. In addition, the waste liquid supplied to the treatment unit 6 may also include the waste liquid generated in the gasification furnace 2. In the first conversion unit 61 in the treatment unit 6, methane is obtained from the waste liquid generated in the reforming furnace 31 and/or the first purification unit 32.
[7B] The methane obtained in the first conversion unit 61 is supplied to the reforming furnace 31 through the gas line GL4 (and the gas line GL1). Thereby, the methane generated in the first conversion unit 61 is converted to a second raw material gas containing carbon monoxide.
[8B] The obtained second raw material gas is purified by the first purification unit 32 and supplied to the culture tank 4.

While the configuration and usage method of the production system 1000 according to Embodiment 2 have been described above, in the production system 1000, the waste liquid generated in the gasification furnace 2 and the raw material gas generation unit 3 is recycled again using the reforming furnace 31 or the like, and thus it can be said that the organic substance production efficiency can be improved.

### <Embodiment 3>

Next, a production system for an organic substance according to Embodiment 3 will be described.

The production system for an organic substance according to Embodiment 3 will be described below, focusing on the differences from the production system for an organic substance according to Embodiment 1 and the production system for an organic substance according to Embodiment 2, and the same points will not be described.

Fig. 5 is a schematic view showing a configuration of a production system for an organic substance according to Embodiment 3.

In the production device 1 of Embodiment 3, the treatment unit 6 includes a second conversion unit 62 and a third conversion unit 63 in addition to the raw material gas generation unit 3. Here, the second conversion unit 62 converts at least a part of the waste liquid to methane. The third conversion unit 63 converts at least some of the methane converted by the second conversion unit 62 to carbon monoxide.

That is, in Embodiment 2, the methane generated in the first conversion unit 61 is transferred to the reforming furnace 31 to obtain a raw material gas containing carbon monoxide, but in Embodiment 3, the treatment unit 6 includes the third conversion unit 63 that can convert methane to carbon monoxide.

The gas containing carbon monoxide (second raw material gas) generated in the third conversion unit 63 may be directly supplied to the culture tank 4 through a gas line GL5 or may be subjected to a purification treatment before it is supplied to the culture tank 4.

That is**,** the treatment unit 6 may further include a second purification unit 64 in addition to the raw material gas generation unit 3. The second purification unit 64 may purify the gas obtained in the third conversion unit 63**.**

The second conversion unit 62 in Embodiment 3 may have the same configuration as the first conversion unit 61 in Embodiment 2. In addition, the third conversion unit 63 may have the same configuration as the reforming furnace 31, or may have a configuration that allows various treatments to be performed to convert methane to carbon monoxide. The treatments include a heat treatment and a chemical and/or a biological treatment.

In addition, the second purification unit 64 may include at least a part of various components contained in the first purification unit 32. On the other hand, the second purification unit 64 may include all of the various components included in the first purification unit 32.

When the second purification unit 64 has a plurality of types of purification components, the disposition order thereof can be appropriately set according to the gas composition supplied from the third conversion unit 63 and the like.

Next, a method of using a production system 100 according to Embodiment 3 (a method for producing an organic substance) will be described.
[1C] First, the exhaust gas discharged from the gasification furnace 2 is supplied to the reforming furnace 31.
[2C] Then, the exhaust gas passed through the reforming furnace 31 is supplied to the reduction unit 321 through the gas line GL3. In the reduction reactor 321a, which is an example of the reduction unit 321, the catalyst 321R reduces carbon dioxide by the contact with carbon dioxide and converts it to carbon monoxide. In this case, the catalyst 321R is oxidized by the contact with carbon dioxide.

Here, the temperature of the reduction reactor 321a (exhaust gas, the catalyst 321R) is preferably 125°C or higher and 500°C or lower, more preferably 150°C or higher and 450°C or lower, and still more preferably 175°C or higher and 400°C or lower.

In addition, the pressure of the reduction reactor 321a (exhaust gas, the catalyst 321R) is preferably less than 1 MPaG, more preferably 0.9 MPaG or less, and still more preferably 0.2 MPaG or more and 0.8 MPaG or less.

When the reaction conditions are set within the above ranges, for example, it is possible to proceed the reduction reaction of carbon dioxide in the reduction reactor 321a more smoothly.

[3C] Next, the exhaust gas passed through the reduction unit 321 is supplied to the washing dehumidifier 322.
[4C] The exhaust gas passed through the washing dehumidifier 322 is supplied to the culture tank 4 as a first raw material gas.
[5C] Finally, the organic substance-containing solution generated in the culture tank 4 is supplied to the recovery unit 5.
[6C] On the other hand, the waste liquid generated in the reforming furnace 31 and/or the first purification unit 32 is supplied to the treatment unit 6 through the liquid line LL2. In addition, the waste liquid supplied to the treatment unit 6 may also include the waste liquid generated in the gasification furnace 2. In the second conversion unit 62 in the treatment unit 6, methane is obtained from the waste liquid generated in the reforming furnace 31 and/or the first purification unit 32.
[7C] The methane obtained in the first conversion unit 61 is supplied to the third conversion unit 63. Thereby, the methane generated in the second conversion unit 62 is converted to a second raw material gas containing carbon monoxide.
[8C] The obtained second raw material gas is purified by the second purification unit 64 and is supplied to the culture tank 4 through the gas line GL5.

While the configuration and usage method of the production system 1000 according to Embodiment 3 have been described above, in the production system 1000, the waste liquid generated in the gasification furnace 2 and the raw material gas generation unit 3 can be converted to a raw material gas containing carbon monoxide by the treatment unit 6 and supplied to the culture tank 4 in the system, and thus it can be said that the organic substance production efficiency can be improved.

### <Others>

The production system 1000 according to each of the above embodiments may have the following configuration.

That is, the above reduction unit 321 having a configuration including one reduction reactor 321a is shown, but the reduction unit 321 may include two or more reduction reactors. Here, when the reduction unit 321 includes two or more reduction reactors in this manner, a gas switching unit may be provided in the reduction unit 321, and the gas supplied from the reforming furnace 31 can be distributed depending on the degree of use of the reduction reactor.

In addition, the reduction unit 321 may include an emission device that emits microwaves.

When an emission device is provided in this manner, it is possible to locally (preferentially) activate only the vicinity of the surface of the reductant (catalyst or the catalyst 321R) with microwaves. Therefore, carbon dioxide can be easily converted to carbon monoxide even at a relatively low temperature.

Microwaves are electromagnetic waves with a frequency of 300 MHz or more and 300 GHz or less, and classified into ultrahigh frequency waves (UHF) with a frequency of 300 MHz or more and 3000 MHz or less, super high frequency waves (SHF) with a frequency of 3 GHz or more and 30 GHz or less, extremely high frequency waves (EHF) with a frequency of 30 GHz or more and 300 GHz or less, and submillimeter waves (SHF) with a frequency of 300 GHz or more and 3000 GHz or less. Among these, the microwaves are preferably ultrahigh frequency waves (UHF). When ultrahigh frequency waves (UHF) are used, the reductant can be heated to a desired temperature in a shorter time.

Here, when microwaves are emitted, an appropriate measure to prevent leakage of radio waves according to the Radio Act is required.

Microwave emission may be performed continuously or intermittently (in a pulsed manner).

Here, the emission device may be disposed outside or inside the reactor (the reduction reactor 321a).

In addition, when the gas composition of the exhaust gas is kept outside the explosion range, it is possible to effectively prevent the exhaust gas from becoming an ignition source regardless of the microwave emission conditions.

### <Embodiment 1'>

Next, a production system for an organic substance according to Embodiment 1' will be described.

A production system for an organic substance according to Embodiment 1' will be described below, focusing on the differences from the production system for an organic substance according to Embodiment 1, and the same points will not be described.

Fig. 6 is a schematic view showing a configuration of the production system for an organic substance according to Embodiment 1'. Fig. 7 is a schematic view showing a configuration of a reduction unit in the production system for an organic substance according to Embodiment 1'.

A production system 100' for an organic substance (hereinafter simply referred to as "production system 100'") shown in Fig. 6 includes a gasification furnace (gas generation unit) 10' and a production device 1' for an organic substance (hereinafter simply referred to as a "production device 1'") connected to the gasification furnace 10'.

Here, in this specification, the upstream side and the downstream side with respect to the flow direction of a gas and a liquid are simply referred to as "upstream" and "downstream," respectively.

In the present embodiment, as the gasification furnace 10', the same furnace exemplified as the gasification furnace 2 can be used. Here, the gas generation unit may be a COₓ emission source of an industrial facility such as that exemplified for the gasification furnace 2 in addition to the gasification furnace 10'.

In each furnace, when the contents are combusted, melted, and refined, an exhaust gas containing carbon dioxide and carbon monoxide (raw material gas) is generated (produced).

The exhaust gas may generally contain other gas components such as hydrogen, nitrogen, oxygen, water vapor, and methane in addition to carbon dioxide and carbon monoxide. The exhaust gas may further contain, as other components, soot, tar, a nitrogen compound, a sulfur compound, a phosphorus compound, an aromatic compound, and the like.

The production device 1' is connected to the gasification furnace 10'. The production device 1' includes a culture tank 2', a purification device 6', a gas line GL1' connecting the gasification furnace 10' and the culture tank 2', and a liquid line LL' connecting the culture tank 2' and the purification device 6'.

In the culture tank 2', an organic substance-containing solution is generated from the supplied exhaust gas (raw material gas) by the action of gas-utilizing bacteria (microbial fermentation).

Gas-utilizing bacteria include both eubacteria and archaebacterial. The eubacteria and archaebacterial are the same as bacteria exemplified in Embodiment 1.

A medium (culture solution) and the culture tank 2' used when gas-utilizing bacteria are cultured may each have the same configuration as the medium (culture solution) and the culture tank 4 described in Embodiment 1.

In order from the side of the gasification furnace 10' (upstream), a reforming furnace 3', a reduction unit 4' (a reduction reactor 4a'), and a pretreatment unit 5' are provided along the gas line GL1'.

Reaction conditions and the like of the reforming furnace 3' may be the same as those of the reforming furnace 31 described in Embodiment 2.

The exhaust gas passed through the reforming furnace 3' is mixed with hydrogen separated in a dehydrogenation device (first separation unit) to be described below and supplied to the reduction reactor 4a' (the reduction unit 4'). In the reduction reactor 4a' in Embodiment 1', carbon monoxide can be generated from carbon dioxide (carbon dioxide can be converted to carbon monoxide) using the hydrogen according to a reverse water gas shift reaction caused by the action of a catalyst.

As shown in Fig. 7, the reduction reactor 4a' is a multi-tubular reaction device (fixed bed type reaction device) including plurality of tubes 41' each filled with (containing) a catalyst 4R' and a housing 42' in which the plurality of tubes 41' are stored in an internal space 43'.

The reduction reactor 4a' (the reduction unit 4') may have the same configuration as the reduction reactor 321a (the reduction unit 321) described in Embodiment 2.

The pretreatment unit 5' includes a dehydrogenation device and a PSA device (separation unit).

The dehydrogenation device (first separation unit) is positioned downstream of the reduction reactor 4a' (the reduction unit 4') and is used to remove (separate) hydrogen from the raw material gas. The dehydrogenation device may be composed of a separator containing a cylindrical separation membrane that selectively permeates and separates hydrogen.

The dehydrogenation device (first separation unit) may have the same configuration as the dehydrogenation device (hydrogen separation unit) 323 described in Embodiment 2.

The dehydrogenation device is connected upstream of the reduction reactor 4a' on the gas line GL1' through the gas line GL2'. With such a configuration, the reformed exhaust gas and hydrogen are mixed and supplied to the reduction reactor 4a'.

In the present embodiment, for example, the reforming furnace 3' and the reduction unit 4' can constitute a first gas purification unit configured to purify an exhaust gas, and the dehydrogenation device can constitute a second gas purification unit configured to purify an exhaust gas.

In this case, the first gas purification unit is provided between the gasification furnace 10' and the PSA device, and the second gas purification unit is provided between the PSA device and the culture tank 2'.

Here, the PSA device may be provided upstream of the first gas purification unit or may be provided downstream of the second gas purification unit.

A removal unit 7' is provided along the gas line GL2'. The removal unit 7' removes impurities that are contained in the gas (before the gas has been supplied to the reduction reactor 4a'), mainly composed of hydrogen, converted from the dehydrogenation device, and that reduce the reactivity of the catalyst (reductant) 4R'.

Such impurities are not particularly limited, and examples thereof include sulfur or sulfur compounds, chlorine or chlorine compounds, and cyanide compounds. Among these, it is preferable to remove sulfur compounds, particularly, hydrogen sulfide, as impurities. By removing hydrogen sulfide, it is possible to effectively prevent the reactivity of the catalyst 4R' from extremely decreasing or being deactivated.

The removal unit 7' may be composed of, for example, a reactor filled with a desulfurizing agent. Examples of desulfurizing agents include an iron oxide-based desulfurizing agent, an activated carbon-based desulfurizing agent, a copper-zinc-based desulfurizing agent, a copper-zinc-aluminum-based desulfurizing agent, and a lime-based desulfurizing agent.

Here, when an iron oxide-based desulfurizing agent is used, since iron sulfide generated by the reaction with hydrogen sulfide can react with oxygen, it is also possible to remove oxygen from the gas that passes through the gas line GL2'.

The PSA device may have the same configuration as the PSA device 324 described in Embodiment 2. If nitrogen is separated from the exhaust gas using the PSA device, it is possible to sufficiently increase the separation efficiency.

The carbon dioxide separated in the PSA device may be mixed with the exhaust gas and supplied to the reduction reactor 4a'.

In addition, the nitrogen separated in the PSA device may be filled into the culture tank 2' when gas-utilizing bacteria are cultured, filled for washing the reduction reactor 4a', filled for washing the PSA device and/or the TSA device (if used), filled for washing a deoxidation device (if used) and/or a deacetylation device (if used), filled for washing pipes in a gas analysis facility, filled for sealing a culture solution storage tank to prevent oxidation of valuables, filled for sealing the purified valuable to prevent oxidation, or filled for washing the facility in a gasification furnace process. Here, nitrogen may be filled into only one of the above devices or filled into two or more of the above devices.

In this manner, when nitrogen is removed from the exhaust gas, the volume of the exhaust gas to be treated downstream can be reduced, and thus the size of the pretreatment unit 5' disposed downstream can be reduced.

As will be described below, the TSA device can be used as one device constituting the second gas purification unit configured to purify an exhaust gas, and according to the type of the device constituting the gas purification unit (first and second gas purification units), nitrogen separated in the PSA device can be filled.

In addition to the dehydrogenation device and the PSA device, the pretreatment unit 5' may include a washing dehumidifier, a filter, a deoxidation device, a deacetylation device, a TSA device, a PTSA device, and the like. Here, these components may be used alone or in any combination, and the order of disposition of these components is arbitrary.

The washing dehumidifier, the filter, the deoxidation device, the deacetylation device, and the TSA device may have the same configuration as the washing dehumidifier 322, the filter, the deoxidation device, the deacetylation device, and the TSA device described in Embodiment 2, respectively.

The PTSA device is a pressure and temperature swing adsorption type separator, and is used, for example, to collectively remove components to be removed by the PSA device and the TSA device. Here, the types of adsorbents and constituent materials used in the TSA device and the PTSA device may be the same as those described in the PSA device.

The exhaust gas treated in the pretreatment unit 5' is supplied to the culture tank 2'.

The concentration of carbon dioxide contained in the exhaust gas supplied to the culture tank 2' is preferably 0.1 vol% or more and 30 vol% or less, more preferably 0.3 vol% or more and 25 vol% or less, still more preferably 0.5 vol% or more and 20 vol% or less, particularly preferably 0.8 vol% or more and 15 vol% or less, and most preferably 1 vol% or more and 10 vol% or less.

The concentrations of carbon monoxide, hydrogen, and nitrogen contained in the exhaust gas supplied to the culture tank 2' are the same as those described in Embodiment 1.

According to the above configuration, since an exhaust gas with a low hydrogen concentration is supplied to the culture tank 2', hydrogen is less likely to reduce the activity of gas-utilizing bacteria. In addition, when the exhaust gas passes through the reduction reactor 4a', since the concentration of carbon monoxide increases, it is possible to efficiently produce an organic substance in the culture tank 2'.

In the culture tank 2', by the action of gas-utilizing bacteria, an organic substance-containing solution is generated from the exhaust gas from which predetermined gas components such as hydrogen, nitrogen, and carbon dioxide are removed (separated).

The purification device 6' is connected to the culture tank 2' through the liquid line LL'. The purification device 6' is a device configured to purify the organic substance from the organic substance-containing solution.

The purification device 6' may have the same configuration as the recovery unit 5 described in Embodiment 1.

The pressure in the distillation device during distillation of the organic substance may be atmospheric pressure, and is preferably less than atmospheric pressure (reduced pressure distillation) and more preferably 60 kPaA or more and 95 kPaA or less. When the pressure is set at such a level, it is possible to improve the organic substance separation efficiency and thus it is possible to improve the yield of the organic substance.

The yield of the organic substance (concentration of the organic substance contained in the purified product) is preferably 90 wt% or more, more preferably 99 wt% or more, and still more preferably 99.5 wt% or more.

The organic substance obtained in this manner is the same as that described in Embodiment 1.

Next, a method of using a production system 100' according to Embodiment 1 (a method for producing an organic substance) will be described.
[1A'] First, the exhaust gas (a raw material gas containing carbon monoxide, carbon dioxide, hydrogen, nitrogen, and other gas components) discharged from the gasification furnace 10' is supplied to the reforming furnace 3'. In this case, methane contained in the exhaust gas is converted to carbon monoxide and hydrogen.
[2A'] Next, the exhaust gas passed through the reforming furnace 3' is mixed with hydrogen separated in the dehydrogenation device.
[3A'] Then, the exhaust gas mixed with hydrogen is supplied to the reduction reactor 4a'. In the reduction reactor 4a', carbon dioxide and hydrogen are used to reduce carbon dioxide and carbon monoxide is generated (reduction step). Specifically, in the reduction reactor 4a', by the action of the catalyst 4R', carbon dioxide and hydrogen are reacted, and converted to carbon monoxide and water. Thereby, the concentration of carbon monoxide and the concentration of water vapor contained in the exhaust gas increase.
[4A'] Next, the exhaust gas passed through the reduction reactor 4a' is supplied to the pretreatment unit 5'. In the pretreatment unit 5', hydrogen is removed (separated) from the exhaust gas by the dehydrogenation device (first separation step).

In addition, the PSA device removes (separates) BTEX, carbon dioxide, nitrogen, and the like from the exhaust gas (separation step). The separated carbon dioxide can be merged with, for example, the gas line GL2'.

In addition, in the pretreatment unit 5', for example, a water-soluble substance, soot, fine particles having a smaller size than soot, oxygen, acetylene, an aromatic compound other than BTEX, and the like may be removed from the exhaust gas.

[5A'] Next, the hydrogen separated in the pretreatment unit 5' passes through the gas line GL2' and merges upstream of the reduction reactor 4a' on the gas line GL1'.

In this case, when the gas mainly composed of hydrogen that passes through the gas line GL2' passes through the removal unit 7', since a sulfur compound (particularly, hydrogen sulfide) is removed, it is possible to prevent or minimize a decrease in the activity of the catalyst 4R' in the reduction reactor 4a'.

[6A'] Next, the exhaust gas passed through the pretreatment unit 5' (exhaust gas from which predetermined gas components such as hydrogen, nitrogen, and carbon dioxide are removed) is supplied to the culture tank 2'. In the culture tank 2', by the action of gas-utilizing bacteria, an organic substance-containing solution is generated from the exhaust gas (culture step).

Here, if the temperature when the organic substance-containing solution is generated in the culture tank 2' is set as X[°C], and the temperature when carbon monoxide is generated in the reduction reactor 4a' (the reduction unit 4') is set as Y[°C], it is preferable to satisfy the relationship of Y≤15X, it is more preferable to satisfy the relationship of Y≤12.5X, and it is still more preferable to satisfy the relationship of Y≤10X. If X and Y satisfy the above relationship, since the temperature when carbon monoxide is generated in the reduction reactor 4a' can be set to be relatively low, there is no need to thoroughly cool the exhaust gas before treatment in the pretreatment unit 5'. Therefore, the amount of heat wasted can be reduced and heat is less likely to be wasted.

In addition, it is preferable to satisfy the relationship of 3X≤Y, it is more preferable to satisfy the relationship of 4X≤Y, and it is still more preferable to satisfy the relationship of 5X≤Y. If X and Y satisfy the above relationship, it is possible to prevent the temperature when carbon monoxide is generated in the reduction reactor 4a' from becoming extremely low and to maintain a sufficient efficiency of converting carbon dioxide to carbon monoxide.

The specific value of X is preferably 25°C or higher and 50°C or lower, more preferably 30°C or higher and 45°C or lower, and still more preferably 35°C or higher and 40°C or lower. The specific value of Y is preferably 125°C or higher and 500°C or lower, more preferably 150°C or higher and 450°C or lower, and still more preferably 175°C or higher and 400°C or lower.

When the organic substance-containing solution is generated in the culture tank 2', the nitrogen removed (separated) by the PSA device may be filled into the culture tank 2'. In this case, the concentration of oxygen contained in the space within the culture tank 2' can be relatively reduced, and adverse effects of oxygen on gas-utilizing bacteria can be prevented or reduced.

[7A'] Finally, the organic substance-containing solution generated in the culture tank 2' is supplied to the purification device 6' through the liquid line LL'. In the purification device 6', the organic substance contained in the organic substance-containing solution is purified to obtain a purified product containing the organic substance at a high concentration.

### <Embodiment 2'>

Next, a production system for an organic substance according to Embodiment 2' will be described.

A production system for an organic substance according to Embodiment 2' will be described below, focusing on the differences from the production system for an organic substance according to Embodiment 1', and the same points will not be described.

Fig. 8 is a schematic view showing a configuration of a reduction unit and the vicinity thereof in the production system for an organic substance according to Embodiment 2'.

A production system 100 according to Embodiment 2' is the same as the production system 100' according to Embodiment 1' except that the configuration of the reduction unit 4' and the vicinity thereof is different.

The production system 100 shown in Fig. 8 includes a carbon dioxide removal device (second separation unit) 8' configured to separate carbon dioxide from the exhaust gas between the reforming furnace 3' and the pretreatment unit 5' on the gas line GL1'. The carbon dioxide removal device 8' may be composed of, for example, a PTSA device (a pressure and temperature swing adsorption type separator), a low-temperature separation type (cryogenic method) separator, a membrane separation type separator, an amine absorption type separator, an amine adsorption type separator, or the like, in addition to the above PSA device or TSA device.

The reduction unit 4' in Embodiment 2' includes a gas switching unit 44' and two reduction reactors 4b1' and 4b2'. The carbon dioxide removal device 8' is connected to the gas switching unit 44' through the gas line GL11', and the dehydrogenation device is connected to the gas switching unit 44' through the gas line GL2'.

The gas switching unit 44' is connected to the inlet ports of the reduction reactors 4b1' and 4b2' through the two gas lines GL31' and GL32'.

The gas switching unit 44' may include, for example, a branch gas line and a flow path opening and closing mechanism such as a valve provided along the branch gas line.

With such a configuration, when the exhaust gas passes through the gas line GL1', carbon dioxide is separated in the carbon dioxide removal device 8'. A gas mainly containing the separated carbon dioxide (hereinafter referred to as an "oxidizing gas") passes through the gas line GL11', the gas switching unit 44', and the gas lines GL31' and GL32' and is supplied to the reduction reactors 4b1' and 4b2'.

On the other hand, a gas mainly containing hydrogen (hereinafter referred to as a "reducing gas") from the dehydrogenation device passes through the gas line GL2', the gas switching unit 44', and the gas lines GL31' and GL32' and is supplied to the reduction reactors 4b1' and 4b2'.

The reduction reactors 4b1' and 4b2' have the same configuration as the reduction reactor 4a' shown in Fig. 7, and a reducing agent 4R' is stored (filled) in the tube 41' in place of the catalyst 4R'. The reducing agent 4R' may have the same shape, size, configuration, and the like as the catalyst 4R' except that the material of the active part is different.

The reducing agent 4R' contains at least one of a metal and an oxide thereof (oxygen carrier having oxygen ion conductivity). The at least one of a metal and an oxide thereof is not particularly limited as long as it can reduce carbon dioxide.

The at least one of a metal and an oxide thereof preferably includes at least one selected from among metal elements belonging to Group 3 to Group 13, more preferably includes at least one selected from among metal elements belonging to Group 3 to Group 12, still more preferably includes at least one among lanthanum, titanium, vanadium, iron, copper, zinc, nickel, manganese, chromium, and cerium, and particularly preferably includes a metal oxide or composite metal oxide containing iron and/or cerium. These metal oxides are useful because they have particularly favorable efficiency of conversion of carbon dioxide to carbon monoxide.

Here, the volumes of the two reduction reactors 4b1' and 4b2' are set to be approximately equal to each other, and are appropriately set according to the amount of the gas supplied. In addition, the volumes of the two reduction reactors 4b1' and 4b2' may be made different depending on the gas composition, the performance of the reducing agent 4R', and the like.

With the above configuration, when gas lines (flow paths) are switched in the gas switching unit 44', for example, an oxidizing gas can be supplied through the gas line GL31' to the reduction reactor 4b1' containing the reducing agent 4R' before oxidation, and a reducing gas can be supplied through the gas line GL32' to the reduction reactor 4b2' containing the reducing agent 4R' after oxidation.

In this case, the reaction of the following Formula 1 proceeds in the reduction reactor 4b1' and the reaction of the following Formula 2 proceeds in the reduction reactor 4b2'. Here, in the following Formula 1 and Formula 2, a case in which the reducing agent 4R' contains iron oxide (FeOₓ₋₁) is exemplified.

Formula 1: CO₂ + FeOₓ₋₁ → CO + FeOₓ

Formula 2: H₂ + FeOₓ → H₂O + FeOₓ₋₁

Then, when the gas lines are switched in the gas switching unit 44' in the opposite direction, the reaction of Formula 2 can be caused to proceed in the reduction reactor 4b1' and the reaction of Formula 1 can be caused to proceed in the reduction reactor 4b2'.

That is, an oxidizing gas and a reducing gas are supplied while switching to the reduction reactor 4b1' and the reduction reactor 4b2'.

Here, the reactions shown in Formula 1 and Formula 2 are both endothermic reactions. Therefore, it is preferable for the production system 100' to further include a reducing agent heating unit (not shown in Fig. 8) configured to heat the reducing agent 4R' when the reducing agent 4R' is brought into contact with an oxidizing gas and a reducing gas (that is, during the reaction between an oxidizing gas and a reducing gas, and the reducing agent 4R').

When such a reducing agent heating unit is provided, it is easy to maintain the temperature in the reaction between the oxidizing gas and the reducing gas with the reducing agent 4R' at a predetermined temperature, it is possible to effectively prevent or minimize a decrease in the efficiency of conversion of carbon dioxide to carbon monoxide, and it is possible to further promote regeneration of the reducing agent 4R' with the reducing gas.

However, depending on the type of the reducing agent 4R', the reactions shown in Formula 1 and Formula 2 may be exothermic reactions. In this case, it is preferable for the production system 100' to include a reducing agent cooling unit configured to cool the reducing agent 4R' in place of the reducing agent heating unit. When such a reducing agent cooling unit is provided, during the reaction between the oxidizing gas and the reducing gas with the reducing agent 4R', it is possible to effectively prevent deterioration of the reducing agent 4R', it is possible to effectively prevent or minimize a decrease in the efficiency of conversion of carbon dioxide to carbon monoxide, and it is possible to further promote regeneration of the reducing agent 4R' with the reducing gas.

That is, in the production system 100', it is preferable to provide a reducing agent temperature adjusting unit configured to adjust the temperature of the reducing agent 4R' depending on the difference in type (exothermic reaction or endothermic reaction) of the reducing agent 4R'.

Here, the conversion rate of carbon dioxide to carbon monoxide in the reduction reactors 4b1' and 4b2' is preferably 70% or more, more preferably 85% or more, and still more preferably 95% or more. The upper limit of the conversion rate of carbon dioxide to the carbon monoxide is generally about 98%.

Such a conversion rate can be set by adjusting the type of the reducing agent 4R' used, the concentration of carbon dioxide contained in the oxidizing gas, the concentration of hydrogen contained in the reducing gas, the temperature of the reduction reactors 4b1' and 4b2', the flow rates (flow velocities) of the oxidizing gas and the reducing gas to the reduction reactors 4b1' and 4b2', the timing of switching between the oxidizing gas and the reducing gas for the reduction reactors 4b1' and 4b2', and the like.

Gas lines GL41' and GL42' are connected to the outlet ports of the reduction reactors 4b1' and 4b2', and these are merged at a gas merging unit J' to form a gas line GL4'. In addition, along the gas lines GL41' and GL42', valves (not shown) are provided as necessary.

For example, when the opening degree of the valve is adjusted, the passing speed of the oxidizing gas and the reducing gas passing through the reduction reactors 4b1' and 4b2' (that is, the treatment speed of the oxidizing gas with the reducing agent 4R' and the treatment speed of the reducing agent 4R' with the reducing gas) can be set.

The gas line GL4' merges with the gas line GL1' between the carbon dioxide removal device 8' and the pretreatment unit 5'. Thereby, carbon monoxide generated in the reduction reactors 4b1' and 4b2' can be merged with the exhaust gas and supplied to the culture tank 2'.

Therefore, in the exhaust gas supplied to the culture tank 2' through the pretreatment unit 5', it is possible to sufficiently increase the concentration of carbon monoxide. As a result, it is possible to further improve the organic substance generation efficiency in the culture tank 2'.

Next, a method of using a production system 100' according to Embodiment 2' (a method for producing an organic substance) will be described.
[0B'] First, when gas lines (flow paths) are switched in the gas switching unit 44', the gas line GL11' communicates with the reduction reactor 4b1', and the gas line GL2' communicates with the reduction reactor 4b2'.
[1B'] Next, the exhaust gas discharged from the gasification furnace 10' is supplied to the reforming furnace 3' .
[2B'] Then, when the exhaust gas passed through the reforming furnace 3' passes through the carbon dioxide removal device 8', carbon dioxide is removed (separated).
[3B-1'] Next, the gas (oxidizing gas) mainly composed of carbon dioxide separated in the carbon dioxide removal device 8' is supplied to the reduction reactor 4b1' through the gas line GL11'. In the reduction reactor 4b1', the reducing agent 4R' reduces carbon dioxide by the contact with carbon dioxide and converts it to carbon monoxide. In this case, the reducing agent 4R' is oxidized by the contact with carbon dioxide.

Here, the temperature of the reduction reactor 4b1' (exhaust gas, the reducing agent 4R') is preferably 125°C or higher and 500°C or lower, more preferably 150°C or higher and 450°C or lower, and still more preferably 175°C or higher and 400°C or lower.

In addition, the heat source here may be obtained by recycling waste heat from the process (for example, a reducing gas boiler, etc.).

In addition, the pressure of the reduction reactor 4b1' (exhaust gas, the reducing agent 4R') is preferably less than 1 MPaG, more preferably 0.9 MPaG or less, and still more preferably 0.2 MPaG or more and 0.8 MPaG or less.

When the reaction conditions are set within the above ranges, for example, it is possible to proceed the reduction reaction of carbon dioxide in the reduction reactor 4b1' more smoothly.

[3B-2'] On the other hand, the gas (reducing gas) mainly composed of hydrogen is supplied from the dehydrogenation device through the gas line GL2' to the reduction reactor 4b2'. In the reduction reactor 4b2', the oxidized reducing agent 4R is reduced (regenerated) by the contact with hydrogen. In this case, water is generated.

Here, the temperature of the reduction reactor 4b2' (reducing gas, the reducing agent 4R') is preferably 125°C or higher and 500°C or lower, more preferably 150°C or higher and 450°C or lower, and still more preferably 175°C or higher and 400°C or lower.

As above, the heat source here may also be obtained by recycling waste heat from the process (for example, a reducing gas boiler, etc.).

In addition, the pressure of the reduction reactor 4b2' (reducing gas, the reducing agent 4R') is preferably less than 1 MPaG, more preferably 0.9 MPaG or less, and still more preferably 0.2 MPaG or more and 0.8 MPaG or less.

When the reaction conditions are set within the above ranges, for example, it is possible to proceed the reduction reaction of the reducing agent 4R' in the reduction reactor 4b2' more smoothly.

When the step [3B-1'] and the step [3B-2'] are alternately performed on the reduction reactors 4b1' and 4b2', it is possible to alternately convert carbon dioxide to carbon monoxide with the reducing agent 4R' and reduce (regenerate) the oxidized reducing agent 4R' with hydrogen.

While the step [3B-1'] and the step [3B-2'] are alternately performed, nitrogen separated in the PSA device may be supplied to (filled into) the reduction reactors 4b1' and 4b2'. That is, the reduction reactors 4b1' and 4b2' may be purged with nitrogen.

[4B'] Next, the gas passed through the reduction reactors 4b1' and 4b2' is mixed with the exhaust gas that passes through the gas line GL1' through GL41', GL42', and GL4' and then supplied to the pretreatment unit 5'.
[5B'] Next, the exhaust gas passed through the pretreatment unit 5' is supplied to the culture tank 2'.
[6B'] Finally, the organic substance-containing solution generated in the culture tank 2' is supplied to the purification device 6'.

With the production system 100' according to Embodiment 2', the same actions and effects as those of the production system 100' according to Embodiment 1' are obtained.

Particularly, in Embodiment 2', in the two reduction reactors 4b1' and 4b2', according to a reduction reaction of carbon dioxide with the reducing agent (reductant) 4R', at least some of the oxygen elements released from carbon dioxide are taken up by the reducing agent 4R' and thus can be separated within the reduction reaction system. Then, an oxidizing gas and a reducing gas are supplied while switching to the two reduction reactors 4b1' and 4b2'. Therefore, it is difficult for the reaction products, carbon monoxide and water, to coexist in one of the reduction reactors 4b1' and 4b2' (reduction reaction system). Therefore, it is possible to prevent or minimize a decrease in the efficiency of conversion of carbon dioxide to carbon monoxide due to restrictions of chemical equilibrium.

Here, in Embodiment 2', the number of reduction reactors installed may be one, or three or more.

When one reduction reactor is installed, for example, an oxidizing gas and a reducing gas can be alternately supplied while switching to the reduction reactor.

In addition, when three or more reduction reactors are installed, for example, when an oxidizing gas is supplied to one reduction reactor, a reducing gas can be supplied to the remaining reduction reactors continuously or in parallel.

In addition, a gas mainly composed of hydrogen has been described as a representative for reducing (regenerating) the oxidized reducing agent 4R', but a gas containing at least one selected from among a hydrocarbon (for example, methane, ethane, acetylene, etc.) and ammonia can also be used in place of hydrogen or in addition to hydrogen.

For example, when a deacetylation device is used, acetylene separated in the deacetylation device may be mixed into the gas line GL2 and supplied to the reduction reactors 4b1' and 4b2'.

In addition, the reduction unit 4' may include an emission device 49' configured to emit microwaves.

Fig. 9 is a schematic view showing a configuration of a reduction unit including an emission device that emits microwaves.

The reduction unit 4' shown in Fig. 9 includes the reduction reactors 4a', 4b1', and 4b2', and the emission device 49'.

When such an emission device 49' is provided, it is possible to locally (preferentially) activate only the vicinity of the surface of the reductant (catalyst or the reducing agent 4R') with microwaves. Therefore, carbon dioxide can be easily converted to carbon monoxide even at a relatively low temperature.

The emission device 49' and emission conditions are the same as those described in the <Others> section after the production system 1000 according to Embodiment 3.

### <Embodiment 3'>

Next, a production system for an organic substance according to Embodiment 3' will be described.

A production system for an organic substance according to Embodiment 3' will be described below, focusing on the differences from the production systems for an organic substance according to Embodiment 1' and Embodiment 2', and the same points will not be described.

Fig. 10 is a schematic view showing a configuration of a reduction unit in the production system for an organic substance according to Embodiment 3'.

A production system 100' according to Embodiment 3' is the same as the production system 100' according to Embodiment 1' except that the configuration of the reduction unit 4' is different.

The reduction unit 4' in Embodiment 3' is composed of a reduction reactor 4c' (also called a reaction cell, an electrolytic bath, or an electrochemical cell) that uses hydrogen and converts carbon dioxide to carbon monoxide according to an electrochemical reduction reaction.

The reduction reactor 4c' shown in Fig. 10 has the housing 42' and a bipolar membrane 47' that divides the space inside the housing 42' into the left and right.

The bipolar membrane 47' is composed of a bonded component in which a cation exchange layer 471' and an anion exchange layer 472' are bonded.

Liquids (for example, electrolytic solution) 421w' and 422w' are stored in a right space 421' and a left space 422' within the housing 42' divided by the bipolar membrane 47'.

Then, a cathode 45' is immersed in the liquid 421w', and an anode 46' is immersed in the liquid 422w'. The cathode 45' and the anode 46' may each be composed of a catalyst, for example, a copper oxide nanowire with the surface coated with tin dioxide.

In addition, a power source 48' is electrically connected to the cathode 45' and the anode 46'. For the power source 48', it is preferable to use a power source that produces electricity as renewable energy. Thereby, it is possible to further improve energy efficiency in the production of organic substances.

In the reduction reactor 4c', when an exhaust gas (carbon dioxide) is supplied to a liquid 491', carbon monoxide is generated according to a reduction reaction of carbon dioxide by the action of the electrons supplied from the power source 48' and the catalyst.

On the other hand, in the liquid 422w', hydroxide ions are converted to oxygen by the removal of electrons at the anode 46'.

In addition, it is preferable that the pH of the liquid 421w' be set to be near neutral, and the pH of the liquid 422w' be set to be alkaline (about 13).

In the present embodiment, the cathode 45' constitutes a reductant that donates electrons and converts carbon dioxide to carbon monoxide. Here, the cathode 45' may be formed by supporting the catalyst on a current collector, and in this case, the catalyst itself constitutes a reductant.

In addition, when a metal complex is used as the catalyst, the metal complex may be dissolved in the liquid 421w'.

According to the production system for an organic substance, the production device for an organic substance, and the method for producing an organic substance described above, it is possible to increase the concentration of carbon monoxide contained in the exhaust gas, and thus it is possible to produce the organic substance efficiently. In addition, when nitrogen is removed from the exhaust gas, the size of a downstream exhaust gas treatment device can be reduced. Therefore, the organic substance can be produced in a smaller facility. In addition, when a reduction unit is provided, it is possible to further increase the concentration of carbon monoxide contained in the exhaust gas, and thus it is possible to produce the organic substance more efficiently.

In addition, in the present disclosure, the organic substance may be generated from the raw material gas in a reactor in which a catalyst is stored (organic substance generation unit).

Examples of such catalysts include ruthenium, rhodium, manganese, germanium, tantalum, zirconium, niobium, hafnium, lanthanum, cerium, aluminum, magnesium, copper, zinc, silicon, and oxides thereof, and these may be used alone or two or more thereof may be used in combination.

In addition, the following aspects may be provided.

(1) A production system for an organic substance, including:
   a gasification furnace configured to generate an exhaust gas;
   a first raw material gas generation unit configured to generate a first raw material gas containing carbon monoxide from the exhaust gas;
   a culture tank to which the first raw material gas is supplied and in which an organic substance-containing solution is generated by the action of gas-utilizing bacteria;
   a recovery unit configured to recover the organic substance from the organic substance-containing solution supplied from the culture tank; and
   a treatment unit configured to obtain a second raw material gas containing carbon monoxide from a waste liquid generated upstream of the culture tank.
(2) The production system for an organic substance according to (1),
   wherein the first raw material gas generation unit includes a reforming furnace and a first purification unit,
   the reforming furnace converts at least some of methane contained in the exhaust gas into carbon monoxide to generate a first crude gas, and
   the first purification unit purifies the first crude gas to generate the first raw material gas.
(3) The production system for an organic substance according to (2),
   wherein the first purification unit further includes a hydrogen separation unit and a reduction unit,
   the hydrogen separation unit is connected downstream of the reduction unit and separates hydrogen from the first crude gas, and
   the reduction unit reduces carbon dioxide in the first crude gas using the separated hydrogen to generate carbon monoxide.
(4) The production system for an organic substance according to (2) or (3),
   wherein the first purification unit further includes a nitrogen separation unit, and
   the nitrogen separation unit is connected downstream of the reduction unit and separates nitrogen from the first crude gas.
(5) The production system for an organic substance according to any one of (2) to (4),
   wherein the treatment unit obtains the second raw material gas from a waste liquid generated in the reforming furnace and/or the first purification unit.
(6) The production system for an organic substance according to any one of (2) to (5),
   wherein the treatment unit includes a first conversion unit in addition to the first raw material gas generation unit,
   the first conversion unit converts at least a part of the waste liquid to methane, and
   the methane converted by the first conversion unit is transferred to the reforming furnace.
(7) The production system for an organic substance according to any one of (1) to (6),
   wherein the treatment unit includes a second conversion unit and a third conversion unit in addition to the first raw material gas generation unit,
   the second conversion unit converts at least a part of the waste liquid to methane, and
   the third conversion unit converts at least some of the methane converted by the second conversion unit to carbon monoxide.
(8) The production system for an organic substance according to (7),
   wherein the treatment unit further includes a second purification unit in addition to the first raw material gas generation unit, and
   the second purification unit purifies the gas obtained in the third conversion unit.
(9) The production system for an organic substance according to any one of (1) to (8),
   wherein a mixed gas of the first raw material gas and the second raw material gas is supplied to the culture tank.
(10) A production device for an organic substance that is used in connection with a gasification furnace configured to generate an exhaust gas, including:
   a first raw material gas generation unit configured to generate a first raw material gas containing carbon monoxide from the exhaust gas;
   a culture tank to which the first raw material gas is supplied and in which an organic substance-containing solution is generated by the action of gas-utilizing bacteria;
   a recovery unit configured to recover the organic substance from the organic substance-containing solution supplied from the culture tank; and
   a treatment unit configured to obtain a second raw material gas containing carbon monoxide from a waste liquid generated upstream of the culture tank.
(11) A method for producing an organic substance, including:
   a first raw material gas generation step of generating a first raw material gas containing carbon monoxide from an exhaust gas;
   a culture step of the first raw material gas being supplied and generating an organic substance-containing solution by the action of gas-utilizing bacteria;
   a recovery step of recovering the organic substance from the organic substance-containing solution obtained in the culture step; and
   a treatment step of obtaining a second raw material gas containing carbon monoxide from a waste liquid generated upstream of the culture step.

(1A) A production system for an organic substance including a gas generation unit configured to generate a raw material gas containing carbon monoxide, carbon dioxide, and hydrogen, a culture tank to which the raw material gas is supplied and in which an organic substance-containing solution is generated by the action of gas-utilizing bacteria, a first separation unit configured to separate the hydrogen from the raw material gas, and a reduction unit having a reductant that reduces the carbon dioxide using the hydrogen separated in the carbon dioxide and the first separation unit to generate carbon monoxide.
(2A) In the production system for an organic substance according to (1A), the first separation unit is positioned downstream of the reduction unit.
(3A) The production system for an organic substance according to (1A) or (2A) further including a second separation unit configured to separate the carbon dioxide from the raw material gas, wherein the carbon dioxide separated in the second separation unit is supplied to the reduction unit.
(4A) The production system for an organic substance according to any one of (1A) to (3A), further including a removal unit configured to remove impurities that are contained in a gas before it is supplied to the reduction unit and that reduce the reactivity of the reductant.
(5A) In the production system for an organic substance according to any one of (1A) to (4A), the carbon monoxide generated in the reduction unit is merged with the raw material gas and supplied to the fermentation tank.
(6A) In the production system for an organic substance according to any one of (1A) to (5A), the reduction unit includes at least one reactor that can separate at least some of the oxygen elements released from the carbon dioxide within the reduction reaction system according to a reduction reaction of the carbon dioxide with the reductant.
(7A) In the production system for an organic substance according to (6A), the reductant is oxidized when it reduces the carbon dioxide and converts it to the carbon monoxide, and the oxidized reductant is reduced by the contact with the hydrogen.
(8A) In the production system for an organic substance according to (7A), the reduction unit includes the plurality of reactors, and the carbon dioxide and the hydrogen are supplied while switching to each of the reactors.
(9A) In the production system for an organic substance according to any one of (1A) to (8A), the concentration of the hydrogen contained in the raw material gas supplied to the culture tank is 1 vol% or more and 30 vol% or less.
(10A) A production device for an organic substance that is used in connection with a gas generation unit configured to generate a raw material gas containing carbon monoxide, carbon dioxide, and hydrogen, including a culture tank to which the raw material gas is supplied and in which an organic substance-containing solution is generated by the action of gas-utilizing bacteria, a first separation unit configured to separate the hydrogen from the raw material gas, and a reduction unit having a reductant that reduces the carbon dioxide using the hydrogen recovered in the carbon dioxide and the first separation unit to generate carbon monoxide.
(11A) A method for producing an organic substance, including a culture step in which a raw material gas containing carbon monoxide, carbon dioxide, and hydrogen is supplied and an organic substance-containing solution is generated by the action of gas-utilizing bacteria, a first separation step in which the hydrogen is separated from the raw material gas, and a reduction step in which the carbon dioxide is reduced with a reductant using the hydrogen separated in the carbon dioxide and the first separation step to generate carbon monoxide.

(1B) A production system for an organic substance, including a gas generation unit configured to generate a raw material gas containing carbon monoxide and nitrogen, a separation unit configured to separate the nitrogen from the raw material gas, and a culture tank to which the raw material gas from which the nitrogen is separated in the separation unit is supplied and in which an organic substance-containing solution is generated by the action of gas-utilizing bacteria.
(2B) The production system for an organic substance according to (1B) further including a gas purification unit provided between the gas generation unit and the separation unit or between the separation unit and the culture tank, and configured to purify the raw material gas.
(3B) In the production system for an organic substance according to (2B), the nitrogen separated in the separation unit is filled into the gas purification unit.
(4B) In the production system for an organic substance according to any one of (1B) to (3B), the separation unit includes a pressure swing adsorption type separator.
(5B) In the production system for an organic substance according to any one of (1B) to (4B), the nitrogen separated in the separation unit is filled into the culture tank and/or the separation unit.
(6B) The production system for an organic substance according to any one of (1B) to (5B), further including a reduction unit having a reductant that reduces the carbon dioxide to generate carbon monoxide.
(7B) In the production system for an organic substance according to (6B), the nitrogen separated in the separation unit is filled into the reduction unit.
(8B) In the production system for an organic substance according to any one of (1B) to (7B), the carbon monoxide generated in the reduction unit is merged with the raw material gas and supplied to the fermentation tank.
(9B) In the production system for an organic substance according to any one of (1B) to (8B), the reduction unit includes at least one reactor that can separate at least some of the oxygen elements released from the carbon dioxide within the reduction reaction system according to a reduction reaction of the carbon dioxide with the reductant.
(10B) In the production system for an organic substance according to (9B), the reductant is oxidized when it reduces the carbon dioxide and converts it to the carbon monoxide, and the oxidized reductant is reduced by the contact with the hydrogen.
(11B) In the production system for an organic substance according to (10B), the reduction unit includes the plurality of reactors, and the carbon dioxide and the hydrogen are supplied while switching to each of the reactors.
(12B) A production device for an organic substance that is used in connection with a gas generation unit configured to generate a raw material gas containing carbon monoxide and nitrogen, including a separation unit configured to separate the nitrogen from the raw material gas and a culture tank to which the raw material gas from which the nitrogen is separated in the separation unit is supplied and in which an organic substance-containing solution is generated by the action of gas-utilizing bacteria.
(13B) A method for producing an organic substance, including a separation step in which, from a raw material gas containing carbon monoxide and nitrogen, the nitrogen is separated, and a culture step to which the raw material gas from which the nitrogen is separated in the separation step is supplied and in which an organic substance-containing solution is generated by the action of gas-utilizing bacteria.

Of course, the present disclosure is not limited thereto.

As described above, various embodiments according to the present disclosure have been described, but these are only examples, and are not intended to limit the scope of the disclosure. The novel embodiments can be implemented in various other forms, and various omissions, substitutions and changes can be made without departing from the spirit and scope of the disclosure. The embodiments and modifications thereof are included in the spirit and scope of the disclosure, and fall within the disclosures described in the appended claims and equivalents thereof.

For example, the production system for an organic substance and the production device for an organic substance of the present disclosure, with respect to the above embodiments, may each have any other additional configurations, may be replaced with any configuration exhibiting the same function, or may have some configurations that are omitted.

In addition, the method for producing an organic substance of the present disclosure, with respect to the above embodiment, may have any other additional step, may be replaced with any step that exhibits the same function, or may have some steps that are omitted.

In addition, in the present disclosure, the configurations described in Embodiment 1 to Embodiment 3 and Embodiment 1' to Embodiment 3' can be arbitrary appropriately combined.

In addition, in the present disclosure, the dehydrogenation device (hydrogen separation unit) configured to separate hydrogen from an exhaust gas (raw material gas) and/or the reduction unit 4' configured to reduce carbon dioxide and generate carbon monoxide may be omitted.

When the dehydrogenation device (hydrogen separation unit) is omitted, for example, hydrogen generated by a hydrogen generation device that uses water electrolysis, hydrogen generated by a device for electrolyzing a sodium chloride aqueous solution, a petroleum steam reforming device, and a device for producing by-product hydrogen such as an ammonia generating device, and hydrogen contained in a coke oven gas discharged from a coke oven can be used.

### [Reference Signs List]

- 1: Production device
- 2: Gasification furnace
- 3: Raw material gas generation unit
- 4: Culture tank
- 5: Recovery unit
- 6: Treatment unit
- 31: Reforming furnace
- 32: First purification unit
- 61: First conversion unit
- 62: Second conversion unit
- 63: Third conversion unit
- 64: Second purification unit
- 100: Production system
- 321: Reduction unit
- 321A: Tube
- 321B: Internal space
- 321C: Housing
- 321R: Catalyst
- 321a: Reduction reactor
- 322: Washing dehumidifier
- 323: Dehydrogenation device
- 324: PSA device
- 1000: Production system
- GL1: Gas line
- GL2: Gas line
- GL3: Gas line
- GL4: Gas line
- GL5: Gas line
- GL6: Gas line
- LL1: Liquid line
- LL2: Liquid line
- 100': Production system for organic substance
- 10': Gasification furnace
- 1': Production device for organic substance
- 2': Culture tank
- 3': Reforming furnace
- 4': Reduction unit
- 4a': Reduction reactor
- 4b1': Reduction reactor
- 4b2': Reduction reactor
- 4c': Reduction reactor
- 41': Tube
- 42': Housing
- 421': Space
- 421w': Liquid
- 422': Space
- 422w': Liquid
- 43': Internal space
- 44': Gas switching unit
- 45': Cathode
- 46': Anode
- 47': Bipolar membrane
- 471': Cation exchange layer
- 472': Anion exchange layer
- 48': Power source
- 49': Emission device
- 4R': Catalyst, Reducing agent
- 5': Pretreatment unit
- 6': Purification device
- 7': Removal unit
- 8': Carbon dioxide removal device
- GL1': Gas line
- GL11': Gas line
- GL2': Gas line
- GL31': Gas line
- GL32': Gas line
- GL4': Gas line
- GL41': Gas line
- GL42': Gas line
- LL': Liquid line
- J': Gas merging unit

## Claims

1. A production system for an organic substance, comprising:
a gasification furnace configured to generate an exhaust gas;
a first raw material gas generation unit configured to generate a first raw material gas containing carbon monoxide from the exhaust gas;
a culture tank to which the first raw material gas is supplied and in which an organic substance-containing solution is generated by the action of gas-utilizing bacteria;
a recovery unit configured to recover the organic substance from the organic substance-containing solution supplied from the culture tank; and
a treatment unit configured to obtain a second raw material gas containing carbon monoxide from a waste liquid generated upstream of the culture tank.

2. The production system for an organic substance according to claim 1,
wherein the first raw material gas generation unit includes a reforming furnace and a first purification unit,
the reforming furnace converts at least some of methane contained in the exhaust gas into carbon monoxide to generate a first crude gas, and
the first purification unit purifies the first crude gas to generate the first raw material gas.

3. The production system for an organic substance according to claim 2,
wherein the first purification unit further includes a hydrogen separation unit and a reduction unit,
the hydrogen separation unit is connected downstream of the reduction unit and separates hydrogen from the first crude gas, and
the reduction unit reduces carbon dioxide in the first crude gas using the separated hydrogen to generate carbon monoxide.

4. The production system for an organic substance according to claim 2 or 3,
wherein the first purification unit further includes a nitrogen separation unit, and
the nitrogen separation unit is connected downstream of the reduction unit and separates nitrogen from the first crude gas.

5. The production system for an organic substance according to any one of claims 2 to 4,
wherein the treatment unit obtains the second raw material gas from a waste liquid generated in the reforming furnace and/or the first purification unit.

6. The production system for an organic substance according to any one of claims 2 to 5,
wherein the treatment unit includes a first conversion unit in addition to the first raw material gas generation unit,
the first conversion unit converts at least a part of the waste liquid to methane, and
the methane converted by the first conversion unit is transferred to the reforming furnace.

7. The production system for an organic substance according to any one of claims 1 to 6,
wherein the treatment unit includes a second conversion unit and a third conversion unit in addition to the first raw material gas generation unit,
the second conversion unit converts at least a part of the waste liquid to methane, and
the third conversion unit converts at least some of the methane converted by the second conversion unit to carbon monoxide.

8. The production system for an organic substance according to claim 7,
wherein the treatment unit further includes a second purification unit in addition to the first raw material gas generation unit, and
the second purification unit purifies the gas obtained in the third conversion unit.

9. The production system for an organic substance according to any one of claims 1 to 8,
wherein a mixed gas of the first raw material gas and the second raw material gas is supplied to the culture tank.

10. A production device for an organic substance to be used in connection with a gasification furnace configured to generate an exhaust gas, comprising:
a first raw material gas generation unit configured to generate a first raw material gas containing carbon monoxide from the exhaust gas;
a culture tank to which the first raw material gas is supplied and in which an organic substance-containing solution is generated by the action of gas-utilizing bacteria;
a recovery unit configured to recover the organic substance from the organic substance-containing solution supplied from the culture tank; and
a treatment unit configured to obtain a second raw material gas containing carbon monoxide from a waste liquid generated upstream of the culture tank.

11. A method for producing an organic substance, comprising:
a first raw material gas generation step of generating a first raw material gas containing carbon monoxide from an exhaust gas;
a culture step of the first raw material gas being supplied and generating an organic substance-containing solution by the action of gas-utilizing bacteria;
a recovery step of recovering the organic substance from the organic substance-containing solution obtained in the culture step; and
a treatment step of obtaining a second raw material gas containing carbon monoxide from a waste liquid generated upstream of the culture step.
